# EUROPEAN PATENT SPECIFICATION

(11) **EP 1 788 967 B1**
(45) Date of publication and mention of the grant of the patent: **12.06.2013**
(21) Application number: 05814100.3
(22) Date of filing: 12.08.2005
(51) Int. Cl.: A61B 18/20, A61C 1/00

(54) **LASER HANDPIECE ARCHITECTURE AND METHODS**
LASERHANDSTÜCKARCHITEKTUR UND VERFAHREN
ARCHITECTURE DE PIECE A MAIN LASER ET PROCEDES ASSOCIES

(30) Priority: 13.08.2004 US 601416 P; 18.09.2004 US 610760 P
(43) Date of publication of application: 30.05.2007
(73) Proprietor: Biolase, Inc., Irvine, CA 92618 (US)
(72) Inventor: JONES, Jeffrey, W., Robertson, Wyoming 82944 (US); BOUTOUSSOV, Dmitri, Dana Point, California 92629 (US)
(74) Representative: Weber-Bruls, Dorothée
(86) International application number: PCT/US2005/028909
(87) International publication number: WO 2006/036337

(56) References cited:
- WO-A2-2005/070129
- WO-A2-2006/012461
- DE-A1- 3 911 853
- DE-A1- 4 401 989
- US-A- 4 718 417

## Description

### BACKGROUND OF THE INVENTION

### 1. Field of the Invention

The present invention relates generally to electromagnetic energy devices and, more particularly, to cutting, treatment and illumination devices that transmit electromagnetic energy toward target surfaces.

### 2. Description of Related Art

Electromagnetic energy devices are employed in a variety of applications. For example, a simple incandescent light may be used to illuminate an area with electromagnetic energy in a form of visible light. Another form of electromagnetic energy, such as a laser beam, may be used to illuminate an area, to identify a target, or to deliver concentrated energy to a target in order to perform various procedures such as melting, cutting, or the like.
Certain medical devices may deliver electromagnetic energy to a target surface such as, for example, an eye, in order to correct a deficiency in visual acuity. Other medical devices may direct electromagnetic energy toward a surface of a tooth to perform, for example, a cutting operation. Endoscopic devices can be used to enhance visualization of internal parts of, for example, a human body in order to detect and/or remove diseased tissue. Constructions of these devices may vary, while underlying functionalities or goals, including, for example, the provision of efficient operation by supplying optimal illumination without obstructing a user's access or view and/or the provision of reliable operation to ensure reproducibility and favorable procedural results, are often shared.

German patent no. DE 3911853 discloses devices which may be useful for delivering electromagnetic radiation to a surface.

A need exists in the prior art to efficiently and reliably transmit various types of electromagnetic energy to and from target surfaces in order, for example, to enhance visualization and treatments of the target surfaces.

### SUMMARY OF THE INVENTION

The present invention addresses these needs by providing a laser handpiece that connects to an electromagnetic energy base unit (e.g., a laser base unit). The invention herein disclosed comprises, according to an exemplary embodiment, a laser handpiece having an elongate portion that receives laser energy, illumination light, excitation light, spray water, spray air, and cooling air from a connector that connects to the laser base unit. The handpiece further comprises a handpiece tip formed as an extension of the elongate portion, the handpiece tip being capable of directing laser energy to a target surface. An embodiment of the elongate portion comprises a plurality of optical fibers.

As used herein, "optical fiber" refers to any electromagnetic energy (e.g., light) transmitting medium (e.g., fiber) that is able to transmit light from one end of the fiber to another end of the fiber. The light transmission may be passive or it may include one or more light altering elements to influence the way light is emitted from the optical fiber. Optical fibers can be used to transmit any type of light, including visible light, infrared light, blue light, laser light, and the like. Optical fibers may be hollow or solid, and may include one or more reflectors within bodies of the fibers to control transmission and emission of light from the optical fibers.

Another embodiment of the present invention comprises a laser device that includes a laser base unit, a connector that connects to the laser base unit, and a conduit that connects to the connector. Further, a laser handpiece connects to the conduit, the laser handpiece being capable of receiving laser energy, illumination light, excitation light, spray water, spray air, and cooling air from the laser base unit.

An illumination device in accordance with an aspect of the present invention includes a unitary distal end (output portion) and a split proximal end (input portion). As used herein, "distal end" refers to an end of an illumination device that is closest to a target surface, and "proximal end" refers to an end of an illumination device that is closest to a power source or other source of electromagnetic energy. The illumination device can include a plurality of different sized optical fibers depending on a particular application for which the illumination device is utilized. In illustrative embodiments, and as disclosed herein, the proximal end of the illumination device includes three proximal end members configured to accommodate three sets of optical fibers.

Another illumination device in accordance with an additional aspect of the present invention includes a plurality of sets of optical fibers configured to emit electromagnetic energy from the distal end of the illumination device toward a target surface. The device further may include at least one optical fiber configured to receive electromagnetic energy from the target surface and transmit the energy to the proximal end of the illumination device. The electromagnetic energy transmitted to the proximal end of the illumination device can be used as a signal for further analysis.

In another implementation of the present invention, an illumination device includes a handpiece having a reflector. The reflector is constructed to reflect both laser energy, such as light provided by an erbium laser, and visible light, such as blue light, toward a target surface. In an illustrated embodiment, as disclosed herein, the reflector includes a plurality of mirrors to provide enhanced control of the emission of electromagnetic energy from the optical fibers toward a target surface and of the transmission of electromagnetic energy reflected from the target surface back through the illumination device in the opposite direction.

A further aspect of the present invention can comprise a method of analyzing feedback light from a handpiece in order to monitor integrity of optical components. One implementation of the method comprises receiving feedback light and generating an electrical signal according to the feedback light. The implementation further can provide an error indication when the electrical signal exceeds a predetermined threshold.

For purposes of summarizing the present invention, certain aspects, advantages and novel features of the present invention are described herein. Of course, it is to be understood that not necessarily all such aspects, advantages or features will be embodied in any particular embodiment of the present invention. Additional advantages and aspects of the present invention are apparent in the following detailed description and claims that follow.

### BRIEF DESCRIPTION OF THE FIGURES

FIG. 1 is a pictorial diagram of a delivery system capable of transferring electromagnetic energy to a treatment site in accordance with an example of the present invention;
FIG. 2 is a pictorial diagram illustrating detail of a connector according to an example of the present invention;
FIG. 3 is a perspective diagram of an embodiment of module that may connect to a laser base unit and that may accept the connector illustrated in FIG. 2;
FIG. 4 is a front view of the embodiment of the module illustrated in FIG. 3;
FIG. 5 is a cross-sectional view of the module illustrated in FIG. 4, the cross-section being taken along a line 5-5' of FIG. 4;
FIG. 6 is another cross-sectional view of the module illustrated in FIG. 4, the cross-section being taken along a line 6-6' of FIG. 4;
FIG. 7 is a pictorial diagram of an embodiment of the conduit shown in FIG. 1;
FIG. 8 is a partial cut-away diagram of a handpiece tip in accordance with an example of the present invention;
FIG. 8a is a pictorial diagram of detail of the handpiece tip of FIG. 8 illustrating a mixing chamber for spray air and water;
FIG. 9 is a sectional view of a proximal member of FIG. 7 taken along line 9-9' of FIG. 7;
FIG. 10 is a cross-sectional view of a handpiece tip taken along line 10-10' of FIG. 8;
FIG. 11 is a cross-sectional diagram of another embodiment of the handpiece tip taken along the line 10-10' of FIG. 8;
FIG. 12 is a cross-sectional diagram of another embodiment of the laser handpiece tip taken along line 12-12' of FIG. 8; and
FIG. 13 is a flow diagram describing an implementation of a method of analyzing feedback light in order to monitor integrity of optical components.

### DETAILED DESCRIPTION OF THE PRESENT INVENTION

Reference will now be made in detail to the presently preferred embodiments of the invention, examples of which are illustrated in the accompanying drawings. Wherever possible, the same or similar reference numbers are used in the drawings and the description to refer to the same or like parts. It should be noted that the drawings are in simplified form and are not to precise scale. In reference to the disclosure herein, for purposes of convenience and clarity only, directional terms, such as, top, bottom, left, right, up, down, over, above, below, beneath, rear, and front, are used with respect to the accompanying drawings. Such directional terms should not be construed to limit the scope of the invention in any manner.

Although the disclosure herein refers to certain illustrated embodiments, it is to be understood that these embodiments are presented by way of example and not by way of limitation. The intent of the following detailed description, although discussing exemplary embodiments, is to be construed to cover all modifications of the embodiments as may fall within the scope of the invention as defined by the appended claims. It is to be understood and appreciated that the process steps and structures described herein do not cover a complete process flow for operation of laser devices. The present invention may be practiced in conjunction with various techniques that are conventionally used in the art, and only so much of the commonly practiced process steps are included herein as are necessary to provide an understanding of the present invention. The present invention has applicability in the field of laser devices in general. For illustrative purposes, however, the following description pertains to a medical laser device and a method of operating the medical laser device to perform surgical functions.

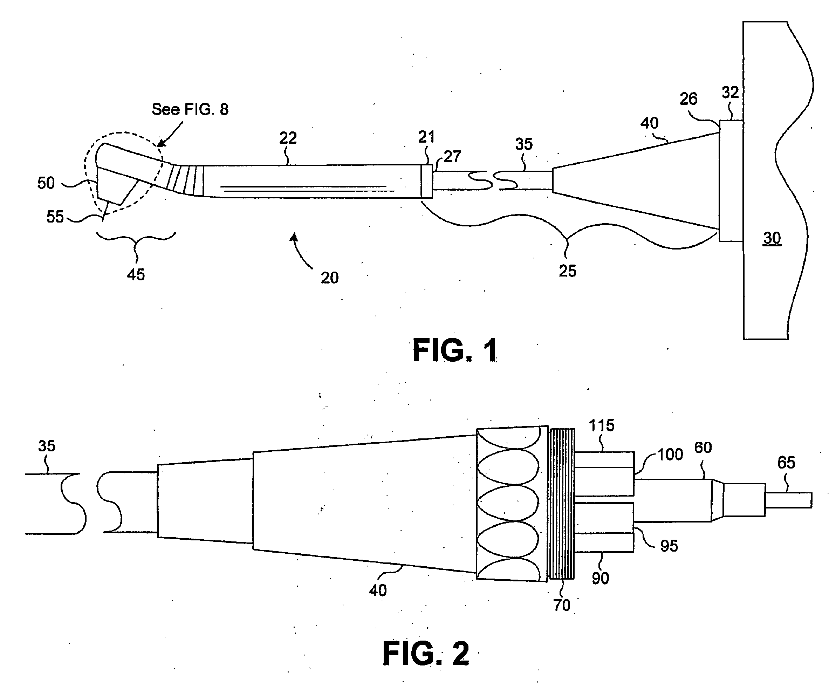

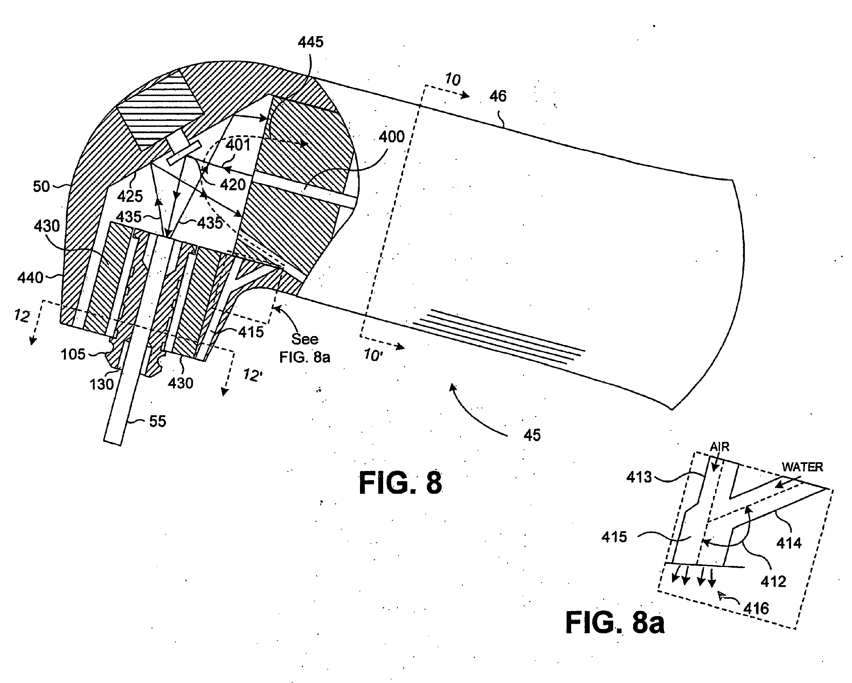

Referring more particularly to the drawings, FIG. 1 is a pictorial diagram of a delivery system capable of transferring laser energy to a treatment site. The illustrated embodiment comprises a laser handpiece 20 that connects to an electromagnetic energy base unit, such as a laser base unit 30, using a linking element 25. The linking element 25 may comprise a conduit 35, which may include one or more optical fibers, tubing for air, tubing for water, and the like. The linking element 25 further may comprise a connector 40 that joins the conduit 35 to the laser base unit 30. The connector 40 may be an identification connector as is described more fully in a U.S. Application No. 11/192,334, publication no. 2006-0142744 filed 7/27/2005 and entitled IDENTIFICATION CONNECTOR FOR A MEDICAL LASER HANDPIECE. The laser handpiece 20 may comprise an elongate portion 22 and a handpiece tip 45 formed as an extension of the elongate portion 22. The elongate portion 22 may have disposed therein a plurality of optical fibers that may connect to, or that are the same as the optical fibers included in the conduit 35. A proximal (i.e., relatively nearer to the laser base unit 30) portion 21 and a distal (i.e., relatively farther from the laser base unit 30) portion 50 may be disposed at respective proximal and distal ends of the laser handpiece 20. The distal portion 50 has protruding therefrom a fiber tip 55, which is described below in more detail with reference to FIG. 8. As illustrated, the linking element 25 has a first end 26 and a second end 27. The first end 26 couples to a receptacle 32 of the laser base unit 30, and the second end 27 couples to the proximal portion 21 of the laser handpiece 20. The connector 40 may connect mechanically to the laser base unit 30 with a threaded connection to the receptacle 32 that forms part of the laser base unit 30.

An embodiment of a connector 40 is illustrated in greater detail in FIG. 2. The illustrated embodiment comprises a laser beam delivery guide connection 60 that may comprise, for example, a treatment optical fiber 65 capable of transmitting laser energy to the laser handpiece 20 (FIG. 1). The illustrated embodiment further comprises a plurality of ancillary connections comprising, in this example, a feedback connection 115, an illumination light connection 100, a spray air connection 95, and a spray water connection 90, that may connect to the laser base unit 30 (FIG. 1). The plurality of ancillary connections further may comprise connections not visible in FIG. 2 such as an excitation light connection and a cooling air connection.

The embodiment of the connector 40 illustrated in FIG. 2 further comprises a threaded portion 70 that may mate with and thereby provide for connection to the receptacle 32 on the laser base unit 30 (FIG. 1).

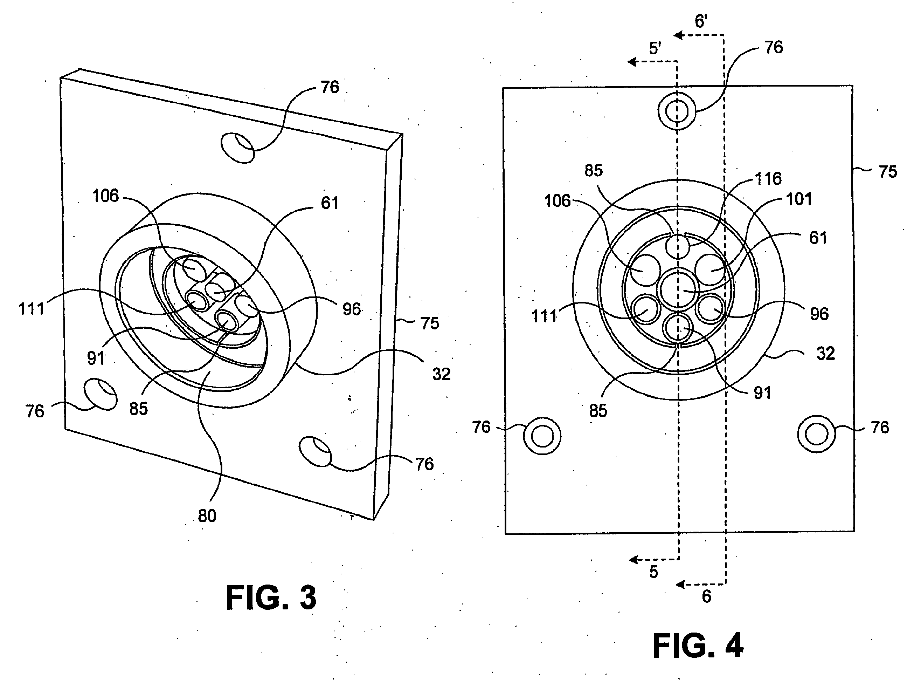

FIG. 3 is a perspective diagram of an embodiment of a module that may connect to, and form a part of, a laser base unit 30 (FIG. 1) and that further may accept connector 40 (FIG. 2). The illustrated embodiment comprises a plate 75 that may fasten to a laser base unit 30 by means of, for example, screws inserted into holes 76. The module comprises a receptacle 32 that may be threaded on an inside surface 80 to mate with threads 70 on the connector 40 (FIG. 2). (Threads are not shown in FIG. 3.) The embodiment of the module further comprises a laser energy coupling 61 mated to the laser beam delivery guide connection 60 (FIG. 2), the laser energy coupling 61 being capable of providing laser energy to the delivery system. The embodiment further comprises a plurality of ancillary couplings including a spray air coupling 96, a spray water coupling 91, a cooling air coupling 111, and an excitation light coupling 106. The embodiment still further comprises a feedback coupling and an illumination light coupling that are not visible in the diagram. One or more key slots 85 may be included to assure that the connector 40 connects to the receptacle 32 in a correct orientation.

FIG. 4 is a front view of the embodiment of the module illustrated in FIG. 3. The view in FIG. 4 illustrates the plate 75 and the holes 76 that may be used to secure the plate module to a laser base unit, such as the laser base unit 30 illustrated in FIG. 1. Further illustrated are the laser energy coupling 61, feedback coupling 116, the illumination light coupling 101, the spray air coupling 96, the spray water coupling 91, the cooling air coupling 111, and the excitation light coupling 106. In operation, the spray water coupling 91 mates with and is capable of supplying spray water to the spray water connection 90 in the connector 40 (FIG. 2). Similarly, the spray air coupling 9 6 mates with and is capable of supplying spray air to the spray air connection 95 in the connector 40. Additionally, the illumination light coupling 101, the excitation light coupling 106, and the cooling air coupling 111 mate with and are capable of supplying, respectively, illumination light to the illumination light connection 100, excitation light to the excitation light connector(not shown), and cooling air to the cooling air connection (not shown) in the connector 40. Further, the feedback coupling 116 mates with and is capable of receiving feedback from the feedback connection 115 in the connector 40. According to an illustrative embodiment, the illumination light coupling 101 and the excitation light coupling 106 couple light from a light-emitting diode (LED) or a laser light source to, respectively, the illumination light connection 100 and the excitation light connection(not shown). One embodiment employs two white LEDs as a source for illumination light. Also illustrated in FIG. 4 are key slots 85 that may prevent the connector 40 from being connected to the receptacle 32 in an incorrect orientation.

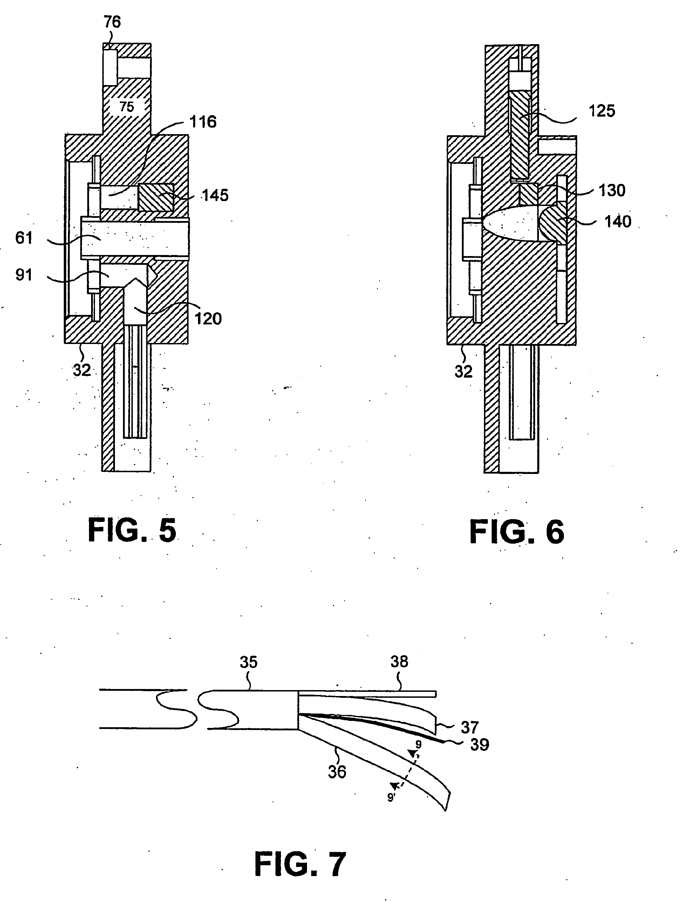

FIG. 5 is a cross-sectional view of the module illustrated in FIGS. 3 and 4. The cross-section is taken along line 5-5' of FIG. 4, the line 5-5' showing cross-sections of the laser energy coupling 61, the feedback coupling 116, and the spray water coupling 91. A water source 120 may supply water to the spray water coupling 91.

FIG. 6 is another cross-sectional view of the module illustrated in FIGS. 3 and 4. The cross-section of FIG 6 is taken along line 6-6' of FIG. 4. The diagram depicts cross-sections of a light source (e.g., an LED 140) that may be capable of supplying light to, for example, one or both of the illumination light coupling 101 (FIG. 4) and the excitation light coupling 106. A pneumatic shutter 125 may control a position of a radiation filter 130 disposed in the laser base unit 30 so that the filter is either inserted or removed from a light path originating with the light source (e.g., the LED 140). For example, one or more pneumatic shutter filters may be provided that enable switching between, for example, blue and white light that is coupled to the illumination light coupling 101 and the excitation light coupling 106 in order to enhance excitation and visualization.

FIG. 7 is a pictorial diagram of an embodiment of the conduit 35 shown in FIG. 1. The illustrated embodiment of the conduit 35 comprises a plurality of proximal members, such as, four proximal members comprising first proximal member 36, second proximal member 37, third proximal member 38, and fourth proximal member 39. First, second, and third proximal members 36, 37, and 38 may have hollow interiors configured to accommodate one or more light transmitters or other tubular or elongate structures that have cross-sectional areas less than a cross-sectional area of a hollow interior of the conduit 35. According to one embodiment, first proximal member 36 comprises an illumination fiber, second proximal member 37 comprises an excitation fiber, and third proximal member 38 comprises a feedback fiber. First, second, and third proximal members 36, 37, and 38 may be arranged such that the hollow interior of each proximal member is in communication with a hollow interior of elongate body 22 (FIG. 1). This arrangement provides for a substantially continuous path for the light transmitters to extend from the proximal portion 21 to the distal portion 50 of the laser handpiece 20. The third proximal member 38 may receive feedback (e.g., reflected or scattered light) from the laser handpiece 20 and may transmit the feedback to the laser base unit 30 as is more particularly described below.

The fourth proximal member 39 may comprise a laser energy fiber that receives laser energy derived from an erbium, chromium, yttrium, scandium, gallium, garnet (Er, Cr:YSGG) solid state laser disposed in the laser base unit 30 (FIG. 1). The laser may generate laser energy having a wavelength of approximately 2.78 microns at an average power of about 6 W, a repetition rate of about 20 Hz, and a pulse width of about 150 microseconds. Moreover, the laser energy may further comprise an aiming beam, such as light having a wavelength of about 655 nm and an average power of about 1 mW transmitted in a continuous-wave (CW) mode. The fourth proximal member 39 may be coupled to or may comprise the treatment optical fiber 65 (FIG. 2) that receives laser energy from the laser energy coupling 61 (FIG. 4). The fourth proximal member 39 further may transmit the laser energy received from the laser base unit 30 to the distal portion 50 of the laser handpiece 20 (FIG. 1).

Although the illustrated embodiment is provided with four proximal members, a greater or fewer number of proximal members may be provided in additional embodiments according to, for example, the number of light transmitters provided by the laser base unit 30. In addition, the illustrated embodiment includes first and second proximal members 36 and 37 that have substantially equal diameters and a third proximal member 38 that has a diameter less than either of the diameters of the first and second proximal members 36 and 37. Other configurations of diameters are also contemplated by the present invention. In an exemplary embodiment, the proximal members connect with the connections in the connector 40 illustrated in FIG. 2. For example, the first proximal member 36 may connect with the illumination light connection 100 and the second proximal member 36 may connect with the excitation light connection (not shown). The third proximal member 38 may connect with the feedback connection 115, and the fourth proximal member 39 may connect with the laser beam delivery guide connection 60 and the treatment optical fiber 65. Attachment of the proximal members 36-39 to the connections may be made internal to connector 40 in a manner known or apparent to those skilled in the art in view of this disclosure and is not illustrated in FIGS. 2 and 7.

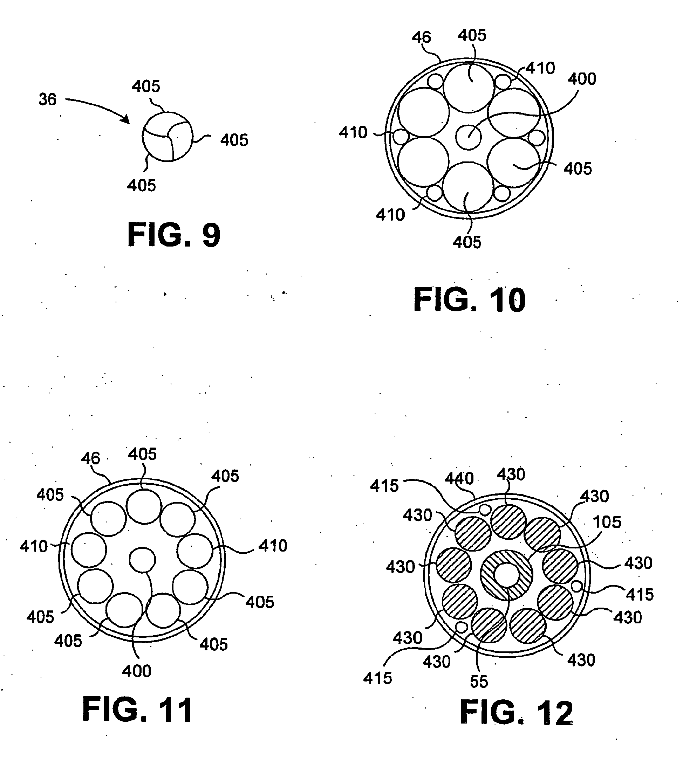

FIG. 8 is a partial cut-away diagram of a handpiece tip 45 (cf. FIG. 1) that couples with the laser base unit 30 by means of the linking element 25 and the elongate portion 22 of the laser handpiece 20. The illustrated embodiment, which is enclosed by an outer surface 46, may receive electromagnetic (e.g., laser) energy, illumination light, excitation light and the like from the laser base unit 30. Typically, the laser energy and light are received by proximal members 36-39 (FIG. 7) as described above and transmitted through waveguides, such as fibers 405 disposed in the elongate portion 22 and the handpiece tip 45 as described below with reference to FIG. 10. For example, illumination light (not shown) may be received by the handpiece tip 45, such as from proximal members 36 and 37 (FIG. 7), carried by fibers 405 (FIG. 10, not shown in FIG. 8), and directed toward a first mirror 425 disposed within the distal portion 50 of the laser handpiece 20. The first mirror 425 in the illustrated embodiment directs illumination light toward a plurality of tip waveguides 430 as is more particularly described below with reference to FIG. 12. Illumination light exiting the tip waveguides 430 may illuminate a target area.

According to one embodiment, concentrated electromagnetic energy, such as laser energy 401, is received (e.g., through fourth proximal member 39 (FIG. 7)) and carried by an internal waveguide such as a treatment optical fiber 400. The laser energy 401 may be directed toward a second mirror 420, which may eclipse at least a part of the first mirror 425 relative to a direction of propagation of the illumination light to the first mirror 425, the second mirror 420 likewise being disposed in the distal portion 50 of the laser handpiece 20. The second mirror 420 may reflect, and thereby direct, the laser energy 401 toward the fiber tip 55. Relative to the concentrated electromagnetic energy (e.g., laser energy 401), the illumination light may comprise an example of additional electromagnetic energy, so described because the illumination light and/or, as described below, excitation light, may comprise electromagnetic energy exhibiting a relatively low power level that is directed to illuminate a portion of a target surface that may, for example, surround a portion of a target surface to which the concentrated electromagnetic energy is directed. The concentrated electromagnetic energy (e.g., laser energy 401) may be directed toward the target surface by the fiber tip 55.

In some embodiments, respective first and second mirrors 425 and 420 may comprise parabolic, toroidal, and/or flat surfaces. FIG. 8 also illustrates a simplified view of a path 445 of cooling air received from a cooling air line (not shown) in the handpiece that may receive cooling air from the cooling air coupling 111 (FIG. 4).

The fiber tip 55 illustrated in FIG. 8 may be encased in a tip ferrule 105 having a distal end. The tip ferrule 105, together with the fiber tip 55, may form a removable, interchangeable unit as is described more fully in U.S. Application No. 11/231, 306, filed 19 September 2005, publication no. US 2007/0128576 and entitled, OUTPUT ATTACHMENTS CODED FOR USE WITH ELECTROMAGNETIC-ENERGY PROCEDURAL DEVICE.

FIG. 9 is a cross-sectional view of first proximal member 36 taken along line 9-9' of FIG. 7 demonstrating that first proximal member 36 (as well as, optionally, second proximal member 37) may comprise three optical fibers 405 substantially fused together to define a unitary light emitting assembly or waveguide. In modified embodiments, the three optical fibers 405 may be joined by other means or not joined. According to other embodiments, one or more of the proximal members, such as the second proximal member 37, can include different numbers of optical fibers 405. In an illustrated embodiment, the second proximal member 37 can include six optical fibers 405 (FIG. 9) that begin to separate and eventually (e.g., at line 10-10' in FIG. 8) surround a laser energy waveguide, such as treatment optical fiber 400, as illustrated in a cross-sectional view of FIG. 10 taken along line 10-10' of FIG. 8 in the handpiece tip 45. In another exemplary embodiment, the second proximal member 37 can include three optical fibers 405 (FIG. 9) and the first proximal member 36 can include three optical fibers 405 (FIG. 9), all six of which begin to separate and eventually (e.g., at line 10-10' in FIG. 8) surround a laser energy waveguide, such as treatment optical fiber 400 in the handpiece tip 45.

The third proximal member 38 may include six relatively smaller fibers 410, as likewise is shown in the cross-sectional view of FIG. 10. Additional waveguides, such as additional fibers 410, may be disposed within the outer surface 46 and, further, may be configured to receive feedback from a target surface. For example, feedback may comprise scattered light 435 (FIG. 8) received from the fiber tip 55 in a manner more particularly described below. The scattered light 435 (i.e., feedback light) may be transmitted by third proximal member 38 (FIG. 7) to the laser base unit 30 (FIG. 1). Fibers 410 are illustrated in FIG. 10 as being separate from each other, but in additional embodiments two or more of the fibers 410 can be fused or otherwise joined together. Fibers 405 and 410 can be manufactured from plastic using conventional techniques, such as extrusion and the like.

FIG. 11 is a cross-sectional diagram of another embodiment of the handpiece tip 45, the cross-section being taken along line 10-10' in FIG. 8. FIG. 11 depicts a laser energy waveguide, such as treatment optical fiber 400 surrounded by illumination waveguides, such as fibers 405, and feedback waveguides, such as fibers 410, all of which are disposed within outer surface 46. In a manner similar to that described above with reference to FIG. 10, the illumination waveguides, such as fibers 405 may receive light energy from the laser base unit 30 (FIG. 1) by way of illumination light coupling 101 (FIG. 4), illumination light connection 100 (FIG. 2), and, for example, proximal members 36 and/or 37 (FIG. 7); and fibers 405 may direct the light to the distal portion 50 of the laser handpiece 20 (FIG. 8).

In certain implementations involving, for example, caries detection, as disclosed in a U.S. Application, filed August 12, 2005 and entitled CARIES DETECTION USING TIMING DIFFERENTAILS BETWEEN EXCITATION AND RETURN PULSES, fibers 405 further may function as both illumination and excitation waveguides. Feedback waveguides, such as fibers 410, may receive feedback light from the fiber tip 55 (FIG. 8) and may transmit the feedback light to third proximal member 38, which couples to or comprises feedback connection 115. The feedback light may be received by the feedback coupling 116, which transmits the light to a feedback detector 145 (FIG. 5) disposed in the laser base unit 30 (FIG. 1). In other embodiments, such as described more fully in the above-referenced U.S. Application No. 11/192,334, publication no. US 2006/0142744, entitled IDENTIFICATION CONNECTOR FOR A MEDICAL LASER HANDPIECE, the laser base unit 30 may additionally supply spray air, spray water, and cooling air to the laser handpiece 20.

FIG. 12 is a cross-sectional diagram of another embodiment of the laser handpiece tip 45 taken along line 12-12' of FIG. 8. This embodiment illustrates a fiber tip 55 surrounded by a tip ferrule or sleeve 105, and, optionally, glue that fills a cavity 130 around the fiber tip 55 to hold the fiber tip 55 in place. Tip waveguides 430 may receive illumination light from second mirror 425 (FIG. 8) and direct the illumination light to a target. In some embodiments, fluid outputs 415, which are disposed in the handpiece tip 45, may carry, for example, air and water. More particularly, illumination light exiting from the illumination fibers 405 (cf. FIG. 11) is reflected by second mirror 425 (FIG. 8) into the tip waveguides 430 (FIGS. 8 and 12). While a portion of this illumination light may also be reflected by second mirror 425 (FIG. 8) into fiber tip 55, fiber tip 55 receives, primarily, a relatively high level of laser energy 401 from treatment optical fiber 400 (cf. FIG. 11), which laser energy, as presently embodied, comprises radiation including both a cutting beam and an aiming beam. In a representative embodiment, illumination light from the illumination fibers 405 that exits the tip waveguides 430 is white light of variable intensity (e.g., adjustable by a user) for facilitating viewing and close examination of individual places of a target surface, such as a tooth. For example, a cavity in a tooth may be closely examined and treated with the aid of light from a plurality of tip waveguides 430.

A detailed illustration of an embodiment of a chamber for mixing spray air and spray water in the handpiece tip 45 is shown in FIG. 8a. As illustrated, the mixing chamber comprises an air intake 413 connected to, for example, tubing (e.g., a spray air line, not shown) that connects to and receives air from, the spray air connection 95 in the connector 40 (FIG. 2). Similarly, a water intake 414 may connect to tubing (also not shown) that connects to and receives water from the spray water connection 90 in the connector 40 (FIG. 2). The air intake 413 and the water intake 414, which may have circular cross-sections about 250 µm in diameter, join at an angle 412 that may approximate 110° in a typical embodiment. Mixing may occur in a neighborhood where the air intake 413 and water intake 414 join, and a spray (e.g., atomized) mixture 416 of water and air may be ejected through a fluid output 415. The embodiment illustrated in FIG. 12 depicts three fluid outputs 415. These fluid outputs may , for example, correspond to, comprise parts of, or comprise substantially all of, any of fluid outputs described in U.S. Application No. 11/042,824, publication no. US 2005/0256517, filed January 24, 2005 and entitled ELECTROMAGNETICALLY INDUCED TREATMENT DEVICES AND METHODS, to the extent compatible, or, in other embodiments, structures described in the referenced provisional patent application may be modified to be compatible with the present invention. The fluid outputs 415 may, as illustrated in FIGS. 8 and 12, have circular cross-sections measuring about 350 µm in diameter.

Scattering of light as described above with reference to FIG. 7 can be detected and analyzed to monitor various conditions. For example, scattering of an aiming beam can be detected and analyzed to monitor, for example, integrity of optical components that transmit the cutting and aiming beams. In typical implementations the aiming beam may cause little to no reflection back into the feedback fibers 410. However, if any components (such as, for example, second mirror 420 or fiber tip 55) is damaged, scattering of the aiming beam light (which may be red in exemplary embodiments) may occur. Scattered light 435 (FIG. 8) may be directed by the second mirror 425 into feedback fibers 410 that may convey the scattered light to the laser base unit 30 (FIG. 1).

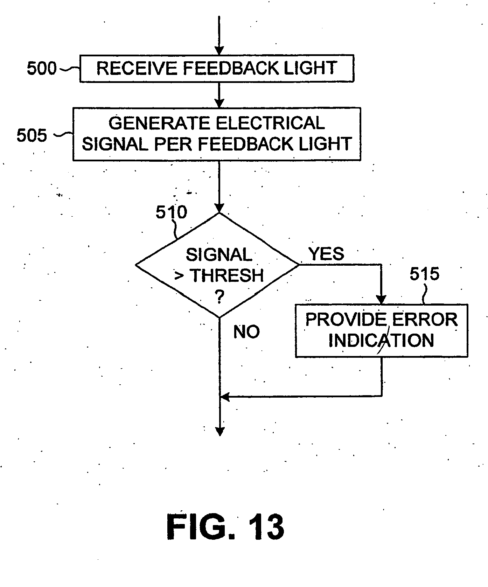

FIG. 13 is a flow diagram describing an implementation of a method of analyzing light, such as feedback light, in order to monitor integrity of optical components. This implementation of the method receives feedback light (i.e., scattered light) at step 500. For example, the feedback light may be received by a light discerning device, such as photo detector 145 (FIG. 5), that forms an electrical signal from the feedback light at step 505. Detection of scattered aiming beam light having an intensity above a predetermined threshold can trigger the laser base unit 30 or other machinery to provide an indication of error or potential error. According to the implementation of the method illustrated in FIG. 13, a magnitude of the electrical signal is compared with the predetermined threshold at step 510. An error indication is provided at step 515 if the electrical signal exceeds the predetermined threshold. That is, a magnitude of detected scattered light 435 from the feedback fibers 410 and/or relative magnitudes of detected scattered light among the various feedback fibers 410 can be automatically analyzed and compared with predetermined optical-component failure criteria to provide additional information to a user regarding a type, location and/or severity of the potential optical-component problem. A feedback display can be provided on a monitor of the laser base unit 30 (e.g., a color of blue) to indicate one or more of the above-described indications or parameters.

The present invention contemplates constructions and uses of visual feedback implements (e.g., cameras) as described in, for example, U.S. Application No. 11/438, 091, filed 18 may 2006, publication no. US 2006/0281042 and entitled ELECTROMAGNETIC RADIATION EMITTING TOOTHBRUSH AND DENTIFRICE SYSTEM, and U.S. Provisional Application No. 60/687,991, filed June 6, 2005 and entitled METHODS FOR TREATING EYE CONDITIONS, on (e.g., attached) or in a vicinity of (e.g., on or near, attached or not, output ends) of electromagnetic energy output devices (e.g., lasers and dental lasers), wherein such output devices, constructions and uses can be, in whole or in part, including any associated methods, modifications, combinations, permutations, and alterations of any constructions(s) or use(s) described or referenced herein or recognizable as included or includable in view of that described or referenced herein by one skilled in the art, to the extent not mutually exclusive, as described in U.S. Application No. 11/033,032, filed January 10, 2005, publication no. US 2006/0241574, and entitled ELECTROMAGNET ENERGY DISTRIBUTIONS FOR ELECTROMAGNETICALLY INDUCED DISRUPTIVE CUTTING, U.S. Application No. 11/033,043, publication no. US 2005/0283143, filed January 10, 2005 and entitled TISSUE REMOVER AND METHOD, U.S. Application No. 11/203, 400, filed 12 August 2005, publication no. US 2006/0142745 and entitled DUAL PULSE-WIDTH MEDICAL LASER WITH PRESETS, U.S. Application No. 11/203,677, filed 12 August 2005, publication no. US 2007/0016176 and entitled LASER HANDPIECE ARCHITECTURE AND METHODS, and U.S. Application No. 09/848,010, filed May 2, 2001 and entitled DERMATOLOGICAL CUTTING AND ABLATING DEVICE. In some embodiments, the sensor may comprise one or more visual feedback implements. The visual feedback implement can be used, for example, (a) in a form that is integrated into a handpiece or output end of an electromagnetic energy output device, (b) in a form that is attached to the handpiece or electromagnetic energy output device, or (c) in conjunction with (e.g., not attached to) the handpiece or electromagnetic energy output device, wherein such handpieces and devices can facilitate cutting, ablating, treatments, and the like. Treatments can include low-level light treatments such as described in the above-referenced U.S. Provisional Application No. 60/687,991 entitled METHODS FOR TREATING EYE CONDITIONS and U.S. Application No. 11/447,605, filed 5 June 2006, publication no. US 2007/0208404, and entitled TISSUE TREATMENT DEVICE AND METHOD.

For example, one implementation may be useful for, among other things, optimizing, monitoring, or maximizing a cutting effect of an electromagnetic energy emitting device, such as a laser handpiece. The laser output can be directed, for example, into fluid (e.g., an air and/or water spray or an atomized distribution of fluid particles from a water connection and/or a spray connection near an output end of the handpiece) that is emitted from the handpiece above a target surface. An apparatus including corresponding structure for directing electromagnetic energy into an atomized distribution of fluid particles above a target surface is disclosed, for example, in the above-referenced U.S. Patent No. 5,574,247. Large amounts of laser energy, for example, can be imparted into the fluid (e.g., atomized fluid particles), which can comprise water, to thereby expand the fluid (e.g., fluid particles) and apply disruptive (e.g., mechanical) cutting forces to the target surface. During a procedure, such as an oral procedure where access and visibility are limited, careful and close-up monitoring by way of a visual feedback implement of (a) interactions between the electromagnetic energy and the fluid (e.g., above the target surface) and/or (b) cutting, ablating, treating or other impartations of disruptive surfaces to the target surface, can improve a quality of the procedure.

In certain embodiments, visualization optical fibers (e.g., a coherent fiber bundle) can be provided that are configured to transmit light from the distal portion 50 to the proximal portion 21, for routing images (e.g., working-surface images) acquired at or in a vicinity of the distal portion by a visual feedback implement. According to some embodiments, the visual feedback implement can comprise an image-acquisition device (e.g., CCD or CMOS camera) for obtaining or processing images from the distal portion. The visual feedback implement can be built-in or attached (e.g., removably attached) to the handpiece and, further, can be disposed at various locations on or in connection with the handpiece between the proximal portion and distal portion, or proximally of the proximal portion. According to this and any of the other embodiments described herein, one or more of the optical fibers described herein and the visualization optical fibers can be arranged, for example, outside of the handpiece envelope. A few applications for the presently-described visual feedback implement may include periodontal pockets (e.g., diagnostic and treatment), endodontics (e.g., visualization of canals), micro-dentistry, tunnel preparations, caries detection and treatment, bacteria visualization and treatment, general dentistry, and airborne-agent and gas detection applications as described in the above-referenced U.S. Application No. 11/438,091.

According to another embodiment of the present invention, electromagnetic radiation (e.g., one or more of blue light, white light, infrared light, a laser beam, reflected/scattered light, fluorescent light, and the like, in any combination) may be transmitted in one or both directions through one or more of the fibers described herein (e.g., feedback, illumination, excitation, treatment), in any combination. Outgoing and incoming beams of electromagnetic radiation can be separated or split, for example, according to one or more characteristics thereof, at the proximal portion or laser base unit using a beam splitter, such as a wavelength-selective beam splitter (not shown), in a manner known to those skilled in the art.

In a representative embodiment, the fluid outputs 415 (FIG. 12) are spaced at zero (a first reference), one hundred twenty, and two hundred forty degrees. In another embodiment, the six illumination/excitation fibers 405 and three feedback fibers 410 (FIG. 11) are optically aligned with and coupled via second mirror 425 on, for example, a one-to-one basis, to nine tip waveguides 430 (FIGS. 8 and12). For example, if nine elements (e.g., six illumination/excitation fibers 405 and three feedback fibers 410) are evenly spaced and disposed at zero (a second reference, which may be the same as or different from the first reference), forty, eighty, one hundred twenty, one hundred sixty, two hundred, two hundred forty, two hundred eighty, and three hundred twenty degrees, then nine tip waveguides 430 may likewise be evenly spaced and disposed at zero, forty, eighty, one hundred twenty, one hundred sixty, two hundred, two hundred forty, two hundred eighty, and three hundred twenty degrees. In another embodiment wherein, for example, the tip waveguides 430 are arranged in relatively closely-spaced groups of three with each group being disposed between two fluid outputs, the tip waveguides 430 may be disposed at, for example, about zero, thirty-five, seventy, one hundred twenty, one hundred fifty-five, one hundred ninety, two hundred forty, two hundred seventy-five, and three hundred ten degrees. In one such embodiment, the tip waveguides 430 may likewise be disposed at about zero, thirty-five, seventy, one hundred twenty, one hundred fifty-five, one hundred ninety, two hundred forty, two hundred seventy-five, and three hundred ten degrees. Further, in such an embodiment, the fluid outputs may be disposed between the groups of tip waveguides at about ninety-five, two hundred fifteen, and three hundred thirty-five degrees.

The cross-sectional views of FIGS. 10 and 11 may alternatively (or additionally), without being changed, correspond to cross-sectional lines 10-10' taken in FIG. 8 closer to (or next to) first and second mirrors 425 and 420 to elucidate corresponding structure that outputs radiation distally onto the first mirror 425 and the second mirror 420. The diameters of illumination/excitation fibers 405 and feedback fibers 410 may be different as illustrated in FIG. 10 or the diameters may be the same or substantially the same as shown in FIG. 11. In an exemplary embodiment, the illumination/excitation fibers 405 and feedback fibers 410 in FIG. 11 comprise plastic constructions with diameters of about 1 mm, and the tip waveguides 430 in FIGS. 8 and 12 comprise sapphire constructions with diameters of about 0.9 mm.

By way of the disclosure herein, a handpiece has been described that utilizes electromagnetic energy to affect a target surface. In the case of dental procedures using laser energy, the handpiece can include an optical fiber for transmitting laser energy to a target surface for treating (e.g., ablating) a dental structure, such as a tooth, a plurality of optical fibers for transmitting light (e.g., blue light) for illumination, curing, whitening, and/or diagnostics of a tooth, a plurality of optical fibers for transmitting light (e.g., white light) to a tooth to provide illumination of the target surface, and a plurality of optical fibers for transmitting light from the target surface back to a sensor for analysis. In the illustrated embodiment, the optical fibers that transmit blue light also transmit white light. In accordance with one aspect of the invention herein disclosed, a handpiece comprises an illumination tube having a feedback signal end and a double mirror handpiece.

In certain embodiments, the methods and apparatuses of the above embodiments can be configured and implemented for use, to the extent compatible and/or not mutually exclusive, with existing technologies including any of the above-referenced apparatuses and methods. Corresponding or related structure and methods are described in the following patents assigned to BioLase Technology, Inc., which corresponding or related structure (and modifications thereof) in the following patents may be (i) operable with, (ii) modified by one skilled in the art to be operable with, and/or (iii) implemented/used with or in combination with any part(s) of, the present invention according to this disclosure, that/those of the patents, and the knowledge and judgment of one skilled in the art: U.S. Patent No. 5,741,247; U.S. Patent No. 5,785,521; U.S. Patent No. 5,968,037; U.S. Patent No. 6,086,367; U.S. Patent No. 6,231,567; U.S. Patent No. 6,254,597, U.S. Patent No. 6, 288,499; U.S. Patent No. 6,350,123; U.S. Patent No. 6,389,193; U.S. Patent No. 6,544,256; U.S. Patent No. 6,561,803; U.S. Patent No. 6,567,582; U.S. Patent No. 6,610,053; U.S. Patent No. 6,616,447; U.S. Patent No. 6,616,451; U.S. Patent No. 6,669,685; and U.S. Patent No. 6,744,790, all of which are commonly assigned.

One implementation may be useful for tailoring, optimizing or maximizing an effect (e.g., cutting or ablating) of a laser. The laser output (e.g., from a power fiber) can be directed, for example, into fluid (e.g., an air and/or water spray or an atomized distribution of fluid particles from a water connection and/or a spray connection near an output end of the handpiece) that is emitted from a fluid output of the handpiece above a target surface (e.g., one or more of tooth, bone, cartilage and soft tissue). The fluid output may comprise a plurality of fluid outputs, concentrically arranged around a power fiber, as described in, for example, U.S. Application No. 11/042,824 referenced above and U.S. Provisional Application No. 60/601,415 referenced above. The power fiber may compri se, for example, a treatment optical fiber, and in various implementations may be coupled to an electromagnetic energy source comprising one or more of a wavelength within a range from about 2.69 to about 2.80 microns and a wavelength of about 2.94 microns. In certain implementations the power fiber may be coupled to one or more of an Er:YAG laser, an Er:YSGG laser, an Er, Cr:YSGG laser and a CTE:YAG laser, and in particular instances may be coupled to one of an Er, Cr:YSGG solid state laser having a wavelength of about 2.789 microns and an Er:YAG solid state laser having a wavelength of about 2.940 microns. An apparatus including corresponding structure for directing electromagnetic energy into an atomized distribution of fluid particles above a target surface is disclosed in the above-referenced U.S. Patent No. 5,574,247, which describes the impartation of laser energy into fluid particles to thereby apply disruptive forces to the target surface.

While this invention has been described with respect to various specific examples and embodiments, it is to be understood that the invention is not limited thereto and that it can be variously practiced. Multiple variations and modification to the disclosed embodiments will occur, to the extent not mutually exclusive, to those skilled in the art upon consideration of the foregoing description. Additionally, other combinations, omissions, substitutions and modifications will be apparent to the skilled artisan in view of the disclosure herein. Accordingly, the present invention should not be limited by the disclosed embodiments, but is to be defined by reference to the appended claims.

## Claims

1. An apparatus, comprising:
a handpiece (20) having a proximal end (21) and a distal end (50), the handpiece being capable of transmitting concentrated infrared electromagnetic energy comprising a second wavelength and relatively less-concentrated visible electromagnetic energy comprising a first wavelength from the proximal end to the distal end, whereby the less-concentrated visible electromagnetic energy is concentrically disposed around the concentrated infrared electromagnetic energy;
a handpiece tip (45) disposed at the distal end of the handpiece (20), the handpiece tip being capable of receiving the concentrated infrared and less-concentrated visible electromagnetic energies from the distal end of the handpiece and of directing the electromagnetic energy to a target;
overlapping reflective surfaces facing about the same direction and being aligned to direct the electromagnetic energies therefrom for routing toward the target, the overlapping reflective surfaces comprising:
a first mirror (425) disposed in a general vicinity between the distal end of the handpiece (20) and the handpiece tip (45) and having a first perimeter surrounding a first reflective surface adapted to reflect the first wavelength, and that is capable of receiving along a first path corresponding to a first direction of travel from the proximal end of the handpiece the first wavelength, and that is capable of directing along a second path corresponding to a second direction of travel toward the handpiece tip the first wavelength for routing along the second path through a first part of the handpiece tip and to the target; and
a second mirror (420) eclipsing at least a part of the first mirror (425), relative to the first direction, the second mirror (420) having a second perimeter which is smaller than the first perimeter and which surrounds a second reflective surface smaller than the first reflective surface, wherein the second reflective surface is adapted to reflect the second wavelength for routing along the second path through a second part of the handpiece tip and to the target, the second mirror (420) being capable of receiving along the first path and directing along the second path concentrated electromagnetic energy, which in the absence of the second mirror (420) would contact the first mirror (425) and not be routed from the second mirror (420) through the second part of the handpiece tip to the target; and
a cooling air line in the handpiece (20) configured to deliver cooling air (445).

2. The apparatus as set forth in Claim 1, wherein the handpiece tip (45) comprises:
a treatment end connected to and disposed at an angle relative to a longitudinal axis of the handpiece (20);
a tip ferrule (105) insertable into the treatment end, the tip ferrule (105) comprising a distal end; and
a fiber tip (55) insertable into the tip ferrule (105), the fiber tip (55) being capable of receiving electromagnetic energy from the distal end of the handpiece (20).

3. The apparatus as set forth in Claim 1, wherein the handpiece (20) comprises:
a first plurality of optical fibers capable of receiving the concentrated infrared and less-concentrated visible electromagnetic energies at the proximal end and of directing the received electromagnetic energies to the handpiece tip (45); and
a second plurality of optical fibers capable of receiving less-concentrated visible electromagnetic energy, which is reflected from a target back into the apparatus, from the handpiece tip (45) and of directing the received less-concentrated visible electromagnetic energy to the proximal end of the handpiece. (20).

4. The apparatus as set forth in Claim 1, wherein during operation the handpiece tip (45) is capable of rotating about an axis of the handpiece (20).

5. The apparatus as set forth in Claim 4, wherein the handpiece tip (45) comprises a plurality of reflectors capable of directing electromagnetic energy from the distal end of the handpiece (20) to a target independent of an angle of rotation of the handpiece tip (45).

6. The apparatus as set forth in Claim 1, in which the handpiece (20) comprises:
an elongate body having a distal end and proximal end;
a power light transmitter;
a first plurality of light transmitters disposed within the elongate body around the power transmitter, the first plurality of light transmitters being configured to transmit electromagnetic energy from the proximal end to the distal end;
a second plurality of light transmitters disposed within, the elongate body around the power transmitter, the second plurality of light transmitters being configured to transmit electromagnetic energy from the distal end to the proximal end; and
a light sensor coupled to receive light from the second plurality of light transmitters at the proximal end.

7. The apparatus as set forth in Claim 6, wherein the second plurality of light transmitters is further configured to transmit light from the distal end to the proximal end.

8. The apparatus as set forth in Claim 6, further comprising a microprocessor coupled to the light sensor to interpret the light received from the second plurality of light transmitters at the proximal end.

9. The apparatus as set forth in Claim 7, wherein the first plurality of light transmitters is capable of transmitting light comprising at least one of visible light, infrared light, blue light, and a laser light.

10. The apparatus as set forth in Claim 7, wherein the elongate body comprises:
a rigid portion; and
at least one substantially flexible portion.

11. The apparatus as set forth in Claim 10, wherein at least one substantially flexible portion comprises a jointed section.

12. The apparatus as set forth in Claim 11, wherein the jointed section assumes in a neutral position an angle of about 15 to 20 degrees with respect to an axis of the rigid portion of the elongate body.

13. The apparatus as set forth in Claim 6, wherein:
the first the plurality of light transmitters comprises a first plurality of optical fibers; and the second plurality of light transmitters comprises a second plurality of optical fibers.

14. The apparatus as set forth in Claim 13, wherein the first plurality of light transmitters comprises at least one light altering element capable of influencing light transmitted to the distal end.

15. The apparatus as set forth in Claim 6, further comprising:
a third plurality of light transmitters extending from the proximal end to the distal end, the third plurality of light transmitters being configured to receive light from the distal end and to direct the light to the proximal end; and
a camera coupled to receive light from at least part of the third plurality of light transmitters.

16. The apparatus as set forth in Claim 15, wherein light transmitted from the distal end to the proximal end by the second plurality of light transmitters is substantially the same as light received by, and directed to the proximal end by, the third plurality of light transmitters.

17. The apparatus as set forth in Claim 6, wherein the apparatus further comprises a camera that is coupled to receive light from the second plurality of light transmitters.

18. The apparatus as set forth in Claim 6, further comprising a beam splitter coupled to one or more of (a) at least part of the first plurality of light transmitters and (b) at least part of the second plurality of light transmitters.

19. The apparatus as set forth in Claim 18, wherein the light sensor is coupled to receive light from part of the second plurality of light transmitters.

20. The apparatus as set forth in Claim 18, further comprising a camera coupled to receive light from at least part of the second plurality of light transmitters.

21. The apparatus as set forth in Claim 1, wherein the handpiece (20) connects to an electromagnetic energy base unit (30), the handpiece comprising an elongate portion (22) coupled to receive concentrated electromagnetic energy and additional electromagnetic energy from a connector (40) that is connectable to the electromagnetic energy base unit (30) wherein the handpiece tip (45) is formed as an extension of the elongate portion and the first minor (425) is disposed in a general vicinity between opposing ends of the handpiece and the elongate portion.

22. The apparatus as set forth in Claim 21, wherein:
the first mirror (425) is capable of receiving additional electromagnetic energy from one or more conduits and directing the additional electromagnetic energy to one or more tip waveguides of the handpiece tip (45), the tip waveguides directing the additional electromagnetic energy toward the target; and
the second mirror (420) is capable of receiving concentrated electromagnetic energy from an electromagnetic energy conduit and directing the concentrated electromagnetic energy to a fiber tip of the handpiece tip, the fiber tip directing the concentrated electromagnetic energy toward the target.

23. The apparatus as set forth in Claim 21, wherein:
the elongate portion is coupled to receive spray water from the connector, and
the elongate portion comprises a spray water line coupled to receive the spray water and to route the spray water through at least part of the elongate portion to the handpiece tip (45).

24. The apparatus as set forth in Claim 21, wherein the additional electromagnetic energy comprises illumination light and excitation light that are both routed through at least part of the elongate portion to the handpiece tip (45).

25. The apparatus as set forth in Claim 24, wherein the illumination light and the excitation light are routed through at least part of the elongate portion by way of an illumination fiber and an excitation fiber.

26. The apparatus as set forth in Claim 24, wherein the elongate handpiece further receives spray water from the connector.

27. The apparatus as set forth in Claim 26 wherein air is further supplied to the elongate handpiece by way of a spray air line.

28. The apparatus as set forth in Claim 21, wherein the elongate portion comprises:
an electromagnetic energy fiber (39) capable of receiving concentrated electromagnetic energy from the connector;
an illumination fiber (36) capable of receiving a portion of the additional electromagnetic energy as illumination light;
an excitation fiber (37) capable of receiving another portion of the additional electromagnetic energy as excitation light; and
a feedback fiber (38) capable of receiving feedback light from the handpiece tip (45).

29. The apparatus as set forth in Claim 28, the first mirror (425) being disposed within the handpiece tip (45) and being capable of directing the additional light as illumination light into a plurality of tip waveguides, the second mirror (420) being disposed within the handpiece tip (45) and being capable of directing the concentrated electromagnetic energy into a fiber tip, and the tip waveguides being capable of receiving illumination and excitation light from the first mirror (425) and directing the illumination light to the target and receiving reflected light from the target and directing the reflected light to the first mirror (425).

30. The apparatus as set forth in Claim 29, further comprising at least one feedback fiber disposed within the handpiece tip (45) and within the elongate portion, wherein the feedback fiber is capable of receiving reflected light from the first mirror (425) and directing the feedback light to the connector.

31. The apparatus as set forth in Claim 30, wherein the electromagnetic energy base unit comprises a photo detector capable of receiving the feedback light from the connector and providing a feedback display according to one of an error condition and potential error condition in optical components of the apparatus.

32. The apparatus as set forth in Claim 21, further comprising:
first tubing disposed within the elongate portion and the handpiece tip (45), the first tubing being capable of receiving air from the connector; and
second tubing disposed within the elongate portion and the handpiece tip (45), the second tubing being capable of receiving water from the connector.

33. The apparatus as set forth in Claim 32, further comprising one or more mixing chambers, each mixing chamber having two inputs and a fluid output, the two inputs comprising:
a first input capable of receiving air from first tubing; and
a second input capable of receiving water from second tubing, wherein the air and water are mixed within the mixing chamber, and a mixture of air and water is expelled from the fluid output.

34. The apparatus as set forth in Claim 32, the one or more mixing chambers comprising three mixing chambers.

35. The apparatus as set forth in Claim 1, the apparatus further comprising:
an electromagnetic energy base unit (30);
a connector (40) that connects to the electromagnetic energy base unit;
a conduit (35) that connects to the connector and the handpiece, wherein the handpiece is capable of receiving the concentrated infrared end less concentrated visible electromagnetic energies as one or more of illumination light and excitation light, and fluid from the electromagnetic energy base unit.

36. The apparatus as set forth in Claim 35, wherein the fluid comprises spray water, spray air, and cooling air.

37. The apparatus as set forth in Claim 35, wherein the handpiece (20) comprises a handpiece tip (45), the handpiece tip comprising:
a plurality of mirrors;
a fiber tip; and
a plurality of tip waveguides, capable of receiving one or more of electromagnetic energy, illumination light, and excitation light, and directing the one or more of electromagnetic energy, illumination light, and excitation light to a target.

38. The apparatus as set forth in Claim 37, wherein the handpiece tip (45) comprises a housing having disposed therein the plurality of tip waveguides, the fiber tip, and a plurality of fluid outputs.

39. The apparatus as set forth in Claim 38, wherein the plurality of tip waveguides and the plurality of fluid outputs are circularly disposed around the fiber tip.

40. The apparatus as set forth in Claim 39, wherein:
the plurality of tip waveguides oomprises nine tip waveguides separated by about forty degrees; and
and the plurality of fluid outputs comprises three fluid outputs separated by about one hundred twenty degrees.

41. The apparatus as set forth in Claim 40, wherein:
the nine tip waveguides are disposed with respect to a reference at zero, forty, eighty, one hundred twenty, one hundred sixty, two hundred, two hundred forty, two hundred eighty, and three hundred twenty degrees; and
the three fluid outputs are disposed with respect to the reference at one hundred, two hundred twenty, and three hundred forty degrees.

42. The apparatus as set forth in Claim 37 wherein the handpiece tip (45) comprises a housing having disposed therein transparent material capable of transmitting light.

43. The apparatus as set forth in Claim 42, wherein the transparent material comprises one of transparent plastic, sapphire, and quartz.

44. The apparatus as set forth in Claim 1, the handpiece (20) comprising:
a power fiber extending from the proximal end to the distal end;
a plurality of first optical fibers concentrically arranged around the power fiber and extending from the proximal end to the distal end, the plurality of first optical fibers being capable of receiving a first type of electromagnetic energy from the proximal end and of outputting the first type of electromagnetic energy at the distal end; and
a plurality of second optical fibers concentrically arranged around the power fiber and extending from the proximal end to the distal end, the plurality of second optical fibers being capable of receiving a second type of electromagnetic energy from the distal end and of directing the second type of electromagnetic energy to the proximal end.

45. The apparatus as set forth in Claim 44, further comprising:
a plurality of third optical fibers extending from the proximal end to the distal end, the plurality of third optical fibers being capable of receiving a third type of electromagnetic energy from the distal end and of directing the third type of electromagnetic energy to the proximal end; and
a camera coupled to receive the third type of electromagnetic energy from at least part of the plurality of third optical fibers.

46. The apparatus as set for this Claim 45, wherein the second type of electromagnetic energy is substantially the same as the third type of electromagnetic energy.

47. The apparatus as set forth in Claim 44, further comprising an electromagnetic energy sensor coupled to receive electromagnetic energy from at least part of the plurality of second optical fibers.

48. The apparatus as set forth in Claim 47, wherein the electromagnetic energy sensor includes a camera coupled to receive electromagnetic energy from at least part of the plurality of second optical fibers.

49. The apparatus set forth in Claim 47, wherein:
the electromagnetic energy sensor is coupled to receive the second type of electromagnetic energy from at least part of the plurality of second optical fibers; and
the handpiece (20) further comprises a camera that is coupled to receive the second type of electromagnetic energy from at least part of the plurality of second optical fibers.

50. The apparatus as set forth in Claim 44, wherein the plurality of first optical fibers is capable of receiving electromagnetic energy comprising one or more of visible light, infrared light, blue light, and laser light.

51. The apparatus as set forth in Claim 44, wherein the apparatus includes at least one light altering element capable of influencing a transmission of electromagnetic energy by the plurality of first optical fibers.

52. The apparatus as set forth in Claim 51, wherein the at least one light altering element comprises at least one optical filter.

53. The apparatus as set forth in Claim 52, wherein the at least one optical filter is structured to convert blue light into white light.

54. The apparatus as set forth in Claim 44, further comprising a beam splitter coupled to one or more of (a) at least part of the plurality of first optical fibers and (b) at least part of the plurality of second optical fibers.

55. A method of analyzing feedback light using the apparatus of Claim 1, thereby monitoring integrity of optical components, the method comprising:
receiving feedback light into the handpiece (20);
generating an electrical signal according to the feedback light; and
providing an error indication when the electrical signal exceeds a predetermined threshold.

56. The method as set forth in Claim 55, wherein the providing of an error indication comprises generating a display on a monitor of an electromagnetic energy base unit.

## Patentansprüche

1. Vorrichtung, welche umfasst:
ein Handstück (20) mit einem proximalen Ende (21) und einem distalen Ende (50), wobei das Handstück konzentrierte infrarote elektromagnetische Energie, welche eine zweite Wellenlänge umfasst, und relativ geringer konzentrierte sichtbare elektromagnetische Energie, welche eine erste Wellenlänge umfasst, vom proximalen Ende zum distalen Ende senden kann, wobei die geringer konzentrierte sichtbare elektromagnetische Energie konzentrisch um die konzentrierte infrarote elektromagnetische Energie angeordnet ist;
eine am distalen Ende des Handstücks (20) angeordnete Handstückspitze (45), wobei die Handstückspitze die konzentrierte infrarote elektromagnetische Energie und die geringer konzentrierte sichtbare elektromagnetische Energie vom distalen Ende des Handstücks empfangen und die elektromagnetische Energie auf ein Ziel lenken kann;
überlappende reflektierende Oberflächen, die in ungefähr die gleiche Richtung zeigen und so ausgerichtet sind, dass die elektromagnetischen Energien von ihnen auf das Ziel gerichtet werden, wobei die überlappenden reflektierenden Oberflächen umfassen:
einen ersten Spiegel (425), der in der Umgebung zwischen dem distalen Ende des Handstücks (20) und der Handstückspitze (45) angeordnet ist und einen ersten Umfang aufweist, der eine erste reflektierende Oberfläche umgibt, die zur Reflexion der ersten Wellenlänge angepasst ist, und der die erste Wellenlänge auf einem ersten Weg empfangen kann, der einer ersten Übertragungsrichtung vom proximalen Ende des Handstücks entspricht, und der die erste Wellenlänge auf einen zweiten Weg lenken kann, der einer zweiten Übertragungsrichtung zur Handstückspitze entspricht, um die erste Wellenlänge auf dem zweiten Weg durch einen ersten Teil der Handstückspitze und auf das Ziel zu richten; und
einen zweiten Spiegel (420), der zumindest einen Teil des ersten Spiegels (425) relativ zur ersten Richtung überlagert, wobei der zweite Spiegel (420) einen zweiten Umfang aufweist, der kleiner als der erste Umfang ist und der eine zweite reflektierende Oberfläche umgibt, die kleiner als die erste reflektierende Oberfläche ist, wobei die zweite reflektierende Oberfläche zur Reflexion der zweiten Wellenlänge angepasst ist, um die zweite Wellenlänge auf dem zweiten Weg durch einen zweiten Teil der Handstückspitze und auf das Ziel zu richten, wobei der zweite Spiegel (420) konzentrierte elektromagnetische Energie auf dem ersten Weg empfangen und auf den zweiten Weg lenken kann, die in Abwesenheit des zweiten Spiegels (420) mit dem ersten Spiegel (425) in Kontakt treten und nicht vom zweiten Spiegel (420) durch den zweiten Teil der Handstückspitze auf das Ziel gerichtet werden würde; und
eine zur Zufuhr von Kühlluft (445) konfigurierte Kühlluftleitung im Handstück (20).

2. Vorrichtung nach Anspruch 1, wobei die Handstückspitze (45) umfasst:
ein mit dem Handstück (20) verbundenes und in einem Winkel relativ zu einer Längsachse des Handstücks (20) angeordnetes Behandlungsende;
eine in das Behandlungsende einsetzbare Spitzenendhülse (105), wobei die Spitzenendhülse (105) ein distales Ende umfasst; und
eine in die Spitzenendhülse (105) einsetzbare Faserspitze (55), wobei die Faserspitze (55) elektromagnetische Energie vom distalen Ende des Handstücks (20) empfangen kann.

3. Vorrichtung nach Anspruch 1, wobei das Handstück (20) umfasst:
eine erste Mehrzahl von Lichtleitfasern, die die konzentrierte infrarote elektromagnetische Energie und die geringer konzentrierte sichtbare elektromagnetische Energie am proximalen Ende empfangen und die empfangene elektromagnetische Energie auf die Handstückspitze (45) lenken können; und
eine zweite Mehrzahl von Lichtleitfasern, die von einem Ziel zurück in die Vorrichtung reflektierte geringer konzentrierte sichtbare elektromagnetische Energie von der Handstückspitze (45) empfangen und die empfangene geringer konzentrierte sichtbare elektromagnetische Energie auf das proximale Ende des Handstücks (20) lenken können.

4. Vorrichtung nach Anspruch 1, wobei die Handstückspitze (45) sich während des Betriebs um eine Achse des Handstücks (20) drehen kann.

5. Vorrichtung nach Anspruch 4, wobei die Handstückspitze (45) eine Mehrzahl von Reflektoren umfasst, welche unabhängig von einem Drehwinkel der Handstückspitze (45) elektromagnetische Energie vom distalen Ende des Handstücks (20) auf ein Ziel lenken können.

6. Vorrichtung nach Anspruch 1, wobei das Handstück (20) umfasst:
einen länglichen Körper mit einem distalen Ende und einem proximalen Ende;
einen leistungsfähigen Lichtsender;
eine erste Mehrzahl von Lichtsendern, die innerhalb des länglichen Körpers um den leistungsfähigen Sender angeordnet sind, wobei die erste Mehrzahl von Lichtsendern zur Übertragung von elektromagnetischer Energie vom proximalen Ende zum distalen Ende konfiguriert sind;
eine zweite Mehrzahl von Lichtsendern, die innerhalb des länglichen Körpers um den leistungsfähigen Sender angeordnet sind, wobei die zweite Mehrzahl von Lichtsendern zur Übertragung von elektromagnetischer Energie vom distalen Ende zum proximalen Ende konfiguriert sind; und
einen Lichtsensor, der zum Empfangen von Licht von der zweiten Mehrzahl von Lichtsendern am proximalen Ende gekoppelt ist.

7. Vorrichtung nach Anspruch 6, wobei die zweite Mehrzahl von Lichtsendern ferner zur Übertragung von Licht vom distalen Ende zum proximalen Ende konfiguriert sind.

8. Vorrichtung nach Anspruch 6, ferner umfassend einen Mikroprozessor, der zum Interpretieren des von der zweiten Mehrzahl von Lichtsendern am proximalen Ende empfangenen Lichts an den Lichtsensor gekoppelt ist.

9. Vorrichtung nach Anspruch 7, wobei die erste Mehrzahl von Lichtsendern Licht senden können, welches sichtbares Licht oder infrarotes Licht oder blaues Licht oder Laserlicht oder Licht mehrerer dieser Bereiche umfasst.

10. Vorrichtung nach Anspruch 7, wobei der längliche Körper umfasst:
einen starren Bereich; und
mindestens einen im Wesentlichen flexiblen Bereich.

11. Vorrichtung nach Anspruch 10, wobei mindestens ein im Wesentlichen flexibler Bereich einen gelenkig verbundenen Abschnitt umfasst.

12. Vorrichtung nach Anspruch 11, wobei der gelenkig verbundene Abschnitt in einer neutralen Stellung einen Winkel von ungefähr 15 bis 20 Grad in Bezug auf eine Achse des starren Bereichs des länglichen Körpers annimmt.

13. Vorrichtung nach Anspruch 6, wobei
die erste Mehrzahl von Lichtsendern eine erste Mehrzahl von Lichtleitfasern umfasst; und die zweite Mehrzahl von Lichtsendern eine zweite Mehrzahl von Lichtleitfasern umfasst.

14. Vorrichtung nach Anspruch 13, wobei
die erste Mehrzahl von Lichtsendern mindestens ein lichtveränderndes Element umfassen, mit dem an das distale Ende gesendetes Licht beeinflussbar ist.

15. Vorrichtung nach Anspruch 6, ferner umfassend:
eine dritte Mehrzahl von Lichtsendern, die sich vom proximalen Ende zum distalen Ende erstrecken, wobei die dritte Mehrzahl von Lichtsendern zum Empfang von Licht vom distalen Ende und zur Lenkung des Lichts zum proximalen Ende konfiguriert sind; und
eine Kamera, die zum Empfangen von Licht von mindestens einem Teil der dritten Mehrzahl von Lichtsendern gekoppelt ist.

16. Vorrichtung nach Anspruch 15, wobei das von der zweiten Mehrzahl von Lichtsendern vom distalen Ende zum proximalen Ende gesendete Licht im wesentlichen dasselbe Licht ist, das von der dritten Mehrzahl von Lichtsendern empfangen und zum proximalen Ende gelenkt wird.

17. Vorrichtung nach Anspruch 6, wobei die Vorrichtung ferner eine Kamera umfasst, die zum Empfangen von Licht von der zweiten Mehrzahl von Lichtsendern gekoppelt ist.

18. Vorrichtung nach Anspruch 6, ferner einen Strahlteiler umfassend, der mindestens an (a) zumindest einen Teil der ersten Mehrzahl von Lichtsendern, oder (b) zumindest einen Teil der zweiten Mehrzahl von Lichtsendern, oder an beide gekoppelt ist.

19. Vorrichtung nach Anspruch 18, wobei der Lichtsensor zum Empfangen von Licht von einem Teil der zweiten Mehrzahl von Lichtsendern gekoppelt ist.

20. Vorrichtung nach Anspruch 18, ferner eine Kamera umfassend, die zum Empfangen von Licht von mindestens einem Teil der zweiten Mehrzahl von Lichtsendern gekoppelt ist.

21. Vorrichtung nach Anspruch 1, wobei das Handstück (20) mit einer elektromagnetische Energie liefernden Basiseinheit (30) verbindbar ist, wobei das Handstück einen länglichen Bereich (22) umfasst, der zum Empfangen von konzentrierter elektromagnetischer Energie und von zusätzlicher elektromagnetischer Energie über ein Anschlussteil (40), das mit der elektromagnetische Energie liefernden Basiseinheit (30) verbindbar ist, gekoppelt ist, wobei die Handstückspitze (45) als eine Verlängerung des länglichen Bereichs gebildet ist und der erste Spiegel (425) in der Umgebung zwischen gegenüberliegenden Enden des Handstücks und dem länglichen Bereich angeordnet ist.

22. Vorrichtung nach Anspruch 21, wobei:
der erste Spiegel (425) zusätzliche elektromagnetische Energie aus einem oder mehreren Leitern empfangen und die zusätzliche elektromagnetische Energie auf eine oder mehrere Wellenleiterspitzen der Handstückspitze (45) lenken kann, wobei die Wellenleiterspitzen die zusätzliche elektromagnetische Energie auf das Ziel lenken; und
der zweite Spiegel (420) konzentrierte elektromagnetische Energie aus einem elektromagnetischen Energieleiter empfangen und die konzentrierte elektromagnetische Energie auf eine Faserspitze der Handstückspitze lenken kann, wobei die Faserspitze die konzentrierte elektromagnetische Energie auf das Ziel lenkt.

23. Vorrichtung nach Anspruch 21, wobei:
der längliche Bereich zur Aufnahme von Sprühwasser vom Anschlussteil gekoppelt ist, und
der längliche Bereich eine Sprühwasserleitung umfasst, die zur Aufnahme des Sprühwassers und zur Weiterleitung des Sprühwassers durch mindestens einen Teil des länglichen Bereichs zur Handstückspitze (45) gekoppelt ist.

24. Vorrichtung nach Anspruch 21, wobei die zusätzliche elektromagnetische Energie Beleuchtungslicht und Anregungslicht umfasst, wobei beide Arten von Licht durch mindestens einen Teil des länglichen Bereichs zur Handstückspitze (45) gelenkt werden.

25. Vorrichtung nach Anspruch 24, wobei das Beleuchtungslicht und das Anregungslicht über eine Beleuchtungsfaser und eine Anregungsfaser durch mindestens einen Teil des länglichen Bereichs gelenkt werden.

26. Vorrichtung nach Anspruch 24, wobei das längliche Handstück ferner Sprühwasser vom Anschlussteil empfängt.

27. Vorrichtung nach Anspruch 26, wobei das längliche Handstück ferner über eine Sprühluftleitung mit Sprühluft versorgt wird.

28. Vorrichtung nach Anspruch 21, wobei der längliche Bereich umfasst:
eine elektromagnetische Energie übertragende Faser (39), die konzentrierte elektromagnetische Energie aus dem Anschlussteil empfangen kann;
eine Beleuchtungsfaser (36), die einen Teil der zusätzlichen elektromagnetischen Energie als Beleuchtungslicht empfangen kann;
eine Anregungsfaser (37), die einen anderen Teil der zusätzlichen elektromagnetischen Energie als Anregungslicht empfangen kann; und
eine Rückmeldungsfaser (38), die Rückmeldungslicht aus der Handstückspitze (45) empfangen kann.

29. Vorrichtung nach Anspruch 28, wobei der erste Spiegel (425) innerhalb der Handstückspitze (45) angeordnet ist und das zusätzliche Licht als Beleuchtungslicht in eine Mehrzahl von Wellenleiterspitzen lenken kann, der zweite Spiegel (420) innerhalb der Handstückspitze (45) angeordnet ist und die konzentrierte elektromagnetische Energie in eine Faserspitze lenken kann, und die Wellenleiterspitzen Beleuchtungslicht und Anregungslicht vom ersten Spiegel (425) empfangen und das Beleuchtungslicht auf das Ziel lenken sowie reflektiertes Licht vom Ziel empfangen und das reflektierte Licht auf den ersten Spiegel (425) lenken können.

30. Vorrichtung nach Anspruch 29, ferner mindestens eine Rückmeldungsfaser umfassend, die innerhalb der Handstückspitze (45) und innerhalb des länglichen Bereichs angeordnet ist, wobei die Rückmeldungsfaser reflektiertes Licht vom ersten Spiegel (425) empfangen und das Rückmeldungslicht auf das Anschlussteil lenken kann.

31. Vorrichtung nach Anspruch 30, wobei die elektromagnetische Energie liefernde Basiseinheit einen Lichtsensor umfasst, der das Rückmeldungslicht vom Anschlussteil empfangen und in Übereinstimmung mit entweder einem Fehlerzustand oder einem möglichen Fehlerzustand in optischen Komponenten der Vorrichtung eine Rückmeldungsanzeige liefern kann.

32. Vorrichtung nach Anspruch 21, ferner umfassend:
eine erste Rohrleitung, die innerhalb des länglichen Bereichs und der Handstückspitze (45) angebracht ist, wobei die erste Rohrleitung Luft aus dem Anschlussteil empfangen kann; und
eine zweite Rohrleitung, die innerhalb des länglichen Bereichs und der Handstückspitze (45) angebracht ist, wobei die zweite Rohrleitung Wasser aus dem Anschlussteil empfangen kann.

33. Vorrichtung nach Anspruch 32, ferner eine oder mehrere Mischkammern umfassend, die jeweils über zwei Eingänge und einen Flüssigkeitsausgang verfügen, wobei die zwei Eingänge umfassen:
einen ersten Eingang, der Luft aus der ersten Rohrleitung empfangen kann; und
einen zweiten Eingang, der Wasser aus der zweiten Rohrleitung empfangen kann, wobei die Luft und das Wasser in der Mischkammer vermischt werden und ein Gemisch aus Luft und Wasser durch den Flüssigkeitsausgang ausgestoßen wird.

34. Vorrichtung nach Anspruch 32, wobei die einen oder mehreren Mischkammern drei Mischkammern umfassen.

35. Vorrichtung nach Anspruch 1, ferner umfassend:
eine elektromagnetische Energie liefernde Basiseinheit (30);
ein Anschlussteil (40), das mit der elektromagnetische Energie liefernden Basiseinheit verbindbar ist;
einen Leiter (35), der mit dem Anschlussteil und dem Handstück verbindbar ist, wobei das Handstück die konzentrierte infrarote elektromagnetische Energie und die geringer konzentrierte sichtbare elektromagnetische Energie in Form von Beleuchtungslicht oder Anregungslicht oder beidem sowie Flüssigkeit aus der elektromagnetische Energie liefernden Basiseinheit empfangen kann.

36. Vorrichtung nach Anspruch 35, wobei die Flüssigkeit Sprühwasser, Sprühluft und Kühlluft umfasst.

37. Vorrichtung nach Anspruch 35, wobei das Handstück (20) eine Handstückspitze (45) umfasst, die Handstückspitze umfassend:
eine Mehrzahl von Spiegeln,
eine Faserspitze; und
eine Mehrzahl von Wellenleiterspitzen, die elektromagnetische Energie oder Beleuchtungslicht oder Anregungslicht oder mehrere dieser Formen empfangen und die elektromagnetische Energie oder Beleuchtungslicht oder Anregungslicht oder mehrere dieser Formen auf ein Ziel lenken können.

38. Vorrichtung nach Anspruch 37, wobei die Handstückspitze (45) ein Gehäuse umfasst, in dem die Mehrzahl von Wellenleiterspitzen, die Faserspitze und eine Mehrzahl von Flüssigkeitsausgängen angeordnet sind.

39. Vorrichtung nach Anspruch 38, wobei die Mehrzahl von Wellenleiterspitzen und die Mehrzahl von Flüssigkeitsausgängen kreisförmig um die Faserspitze angeordnet sind.

40. Vorrichtung nach Anspruch 39, wobei:
die Mehrzahl von Wellenleiterspitzen neun Wellenleiterspitzen umfasst, die jeweils um ungefähr vierzig Grad versetzt sind; und
die Mehrzahl von Flüssigkeitsausgängen drei Flüssigkeitsausgänge umfasst, die jeweils um ungefähr einhundertzwanzig Grad versetzt sind.

41. Vorrichtung nach Anspruch 40, wobei:
die neun Wellenleiterspitzen relativ zu einer Bezugslinie in Winkeln von null, vierzig, achtzig, einhundertzwanzig, einhundertsechzig, zweihundert, zweihundertvierzig, zweihundertachtzig und dreihundertzwanzig Grad angeordnet sind; und
die drei Flüssigkeitsausgänge relativ zu der Bezugslinie in Winkeln von einhundert, zweihundertzwanzig und dreihundertvierzig Grad angeordnet sind.

42. Vorrichtung nach Anspruch 37, wobei die Handstückspitze (45) ein Gehäuse umfasst, in dem transparentes Material angeordnet ist, das Licht übertragen kann.

43. Vorrichtung nach Anspruch 42, wobei das transparente Material entweder transparenten Kunststoff oder Saphir oder Quarz umfasst.

44. Vorrichtung nach Anspruch 1, wobei das Handstück (20) umfasst:
eine Leistungsfaser, die sich vom proximalen Ende zum distalen Ende erstreckt;
eine Mehrzahl von ersten Lichtleitfasern, die konzentrisch um die Leistungsfaser angeordnet sind und sich vom proximalen Ende zum distalen Ende erstrecken, wobei die Mehrzahl von ersten Lichtleitfasern eine erste Form von elektromagnetischer Energie vom proximalen Ende empfangen und die erste Form von elektromagnetischer Energie am distalen Ende ausgeben können; und
eine Mehrzahl von zweiten Lichtleitfasern, die konzentrisch um die Leistungsfaser angeordnet sind und sich vom proximalen Ende zum distalen Ende erstrecken, wobei die Mehrzahl von zweiten Lichtleitfasern eine zweite Form von elektromagnetischer Energie vom distalen Ende empfangen und die zweite Form von elektromagnetischer Energie an das proximale Ende lenken können.

45. Vorrichtung nach Anspruch 44, ferner umfassend:
eine Mehrzahl von dritten Lichtleitfasern, die sich vom proximalen Ende zum distalen Ende erstrecken, wobei die Mehrzahl von dritten Lichtleitfasern eine dritte Form von elektromagnetischer Energie vom distalen Ende empfangen und die dritte Form von elektromagnetischer Energie an das proximale Ende lenken können; und
eine Kamera, die zum Empfangen der dritten Form von elektromagnetischer Energie von mindestens einem Teil der Mehrzahl von dritten Lichtleitfasern gekoppelt ist.

46. Vorrichtung nach Anspruch 45, wobei die zweite Form von elektromagnetischer Energie im Wesentlichen dieselbe ist wie die dritte Form von elektromagnetischer Energie.

47. Vorrichtung nach Anspruch 44, ferner einen elektromagnetischen Energiesensor umfassend, der zum Empfangen von elektromagnetischer Energie von mindestens einem Teil der Mehrzahl von zweiten Lichtleitfasern gekoppelt ist.

48. Vorrichtung nach Anspruch 47, wobei der elektromagnetische Energiesensor eine Kamera enthält, die zum Empfangen von elektromagnetischer Energie von mindestens einem Teil der Mehrzahl von zweiten Lichtleitfasern gekoppelt ist.

49. Vorrichtung nach Anspruch 47, wobei:
der elektromagnetische Energiesensor zum Empfangen der zweiten Form von elektromagnetischer Energie von mindestens einem Teil der Mehrzahl von zweiten Lichtleitfasern gekoppelt ist; und
das Handstück (20) ferner eine Kamera umfasst, die zum Empfangen der zweiten Form von elektromagnetischer Energie von mindestens einem Teil der Mehrzahl von zweiten Lichtleitfasern gekoppelt ist.

50. Vorrichtung nach Anspruch 44, wobei die Mehrzahl von ersten Lichtleitfasern elektromagnetische Energie empfangen kann, welche sichtbares Licht oder infrarotes Licht oder blaues Licht oder Laserlicht oder Licht mehrerer dieser Bereiche umfasst.

51. Vorrichtung nach Anspruch 44, wobei die Vorrichtung mindestens ein lichtveränderndes Element umfasst, mit dem eine Übertragung von elektromagnetischer Energie durch die Mehrzahl von ersten Lichtleitfasern beeinflussbar ist.

52. Vorrichtung nach Anspruch 51, wobei das mindestens eine lichtverändernde Element mindestens ein optisches Filter umfasst.

53. Vorrichtung nach Anspruch 52, wobei das mindestens eine optische Filter so aufgebaut ist, dass es blaues Licht in weißes Licht umwandelt.

54. Vorrichtung nach Anspruch 44, ferner einen Strahlteiler umfassend, der mindestens an (a) zumindest einen Teil der Mehrzahl von ersten Lichtleitfasern, oder (b) zumindest einen Teil der Mehrzahl von zweiten Lichtleitfasern, oder an beide gekoppelt ist.

55. Verfahren zur Analyse von Rückmeldungslicht mittels der Vorrichtung nach Anspruch 1 und damit zur Überwachung der Integrität von optischen Komponenten, umfassend:
das Empfangen von Rückmeldungslicht im Handstück (20);
das Erzeugen eines elektrischen Signals in Übereinstimmung mit dem Rückmeldungslicht; und
das Bereitstellen einer Fehlermeldung, sobald das elektrische Signal einen vorher festgelegten Grenzwert überschreitet.

56. Verfahren nach Anspruch 55, wobei das Bereitstellen einer Fehlermeldung die Erzeugung einer Anzeige auf einem Bildschirm einer elektromagnetische Energie liefernden Basiseinheit umfasst.

## Revendications

1. Appareil comprenant :
une pièce à main (20) ayant une extrémité proximale (21) et une extrémité distale (50), la pièce à main pouvant transmettre une énergie électromagnétique d'infrarouge concentrée comprenant une seconde longueur d'onde et une énergie électromagnétique visible relativement moins concentrée comprenant une première longueur d'onde à partir de l'extrémité proximale vers l'extrémité distale, de sorte que l'énergie électromagnétique visible moins concentrée est disposée concentriquement autour de l'énergie électromagnétique d'infrarouge concentrée ;
un embout de pièce à main (45) disposé à l'extrémité distale de la pièce à main (20), l'embout de pièce à main étant capable de recevoir les énergies électromagnétiques d'infrarouge concentrée et visible moins concentrée provenant de l'extrémité distale de la pièce à main et de diriger l'énergie électromagnétique vers une cible ;
des surfaces réflectrices se recouvrant dirigées environ dans la même direction et alignées pour diriger les énergies électromagnétiques provenant de celles-ci pour acheminement vers la cible, les surfaces réflectrices se recouvrant comprenant :
un premier miroir (425) disposé dans un voisinage général entre l'extrémité distale de la pièce à main (20) et l'embout de pièce à main (45) et ayant un premier périmètre entourant une première surface réflectrice adaptée pour réfléchir la première longueur d'onde, et qui est capable de recevoir le long d'un premier trajet correspondant à une première direction de déplacement à partir de l'extrémité proximale de la pièce à main la première longueur d'onde, et qui est capable de diriger le long d'un second trajet correspondant à une seconde direction de déplacement vers l'embout de pièce à main la première longueur d'onde pour acheminement le long du second trajet à travers une première partie de l'embout de pièce à main et jusqu'à la cible ; et
un second miroir (420) éclipsant au moins une partie du premier miroir (425), par rapport à la première direction, le second miroir (420) ayant un second périmètre qui est plus petit que le premier périmètre et qui entoure une seconde surface réflectrice plus petite que la première surface réflectrice, dans lequel la seconde surface réflectrice est adaptée pour réfléchir la seconde longueur d'onde pour acheminement le long du second trajet à travers une seconde partie de l'embout de pièce à main et vers la cible, le second miroir (420) étant capable de recevoir le long du premier trajet et diriger le long du second trajet une énergie électromagnétique concentrée, qui en l'absence du second miroir (420) viendrait en contact avec le premier miroir (425) et ne serait pas acheminée à partir du second miroir (420) à travers la seconde partie de l'embout de pièce à main vers la cible ; et
une ligne d'air de refroidissement située dans la pièce à main (20) configurée pour fournir de l'air de refroidissement (445).

2. Appareil selon la revendication 1, dans lequel l'embout de pièce à main (45) comprend :
une extrémité de traitement reliée à la pièce à main et disposée selon un angle par rapport à un axe longitudinal de la pièce à main (20) ;
une bague d'embout (105) pouvant être insérée dans l'extrémité de traitement, la bague d'embout (105) comprenant une extrémité distale ; et
un embout de fibre (55) pouvant être inséré dans la bague d'embout (105), l'embout de fibre (55) pouvant recevoir une énergie électromagnétique provenant de l'extrémité distale de la pièce à main (20).

3. Appareil selon la revendication 1, dans lequel la pièce à main (20) comprend :
une première pluralité de fibres optiques capables de recevoir les énergies électromagnétiques d'infrarouge concentrée et visible moins concentrée au niveau de l'extrémité proximale et de diriger les énergies électromagnétiques reçues vers l'embout de pièce à main (45) ; et
une deuxième pluralité de fibres optiques capables de recevoir de l'énergie électromagnétique visible moins concentrée, qui est réfléchie à partir d'une cible en retour dans l'appareil, à partir de l'embout de pièce à main (45) et de diriger l'énergie électromagnétique visible moins concentrée reçue vers l'extrémité proximale de la pièce à main (20).

4. Appareil selon la revendication 1, dans lequel pendant un fonctionnement l'embout de pièce à main (45) est capable de tourner autour d'un axe de la pièce à main (20).

5. Appareil selon la revendication 4, dans lequel l'embout de pièce à main (45) comprend plusieurs réflecteurs capables de diriger une énergie électromagnétique depuis l'extrémité distale de la pièce à main (20) vers une cible indépendamment de l'angle de rotation de l'embout de pièce à main (45).

6. Appareil selon la revendication 1, dans lequel la pièce à main (20) comprend :
un corps allongé ayant une extrémité distale et une extrémité proximale ;
et un émetteur de lumière de puissance ;
une première pluralité d'émetteurs de lumière disposée dans le corps allongé autour de l'émetteur de puissance, pour transmettre une énergie électromagnétique depuis l'extrémité proximale vers l'extrémité distale ;
une deuxième pluralité d'émetteurs de lumière disposée dans le corps allongé autour de l'émetteur de puissance, la deuxième pluralité d'émetteurs de lumière étant configurée pour transmettre une énergie électromagnétique depuis l'extrémité distale vers l'extrémité proximale ; et
un détecteur de lumière couplé pour recevoir une lumière provenant de la deuxième pluralité d'émetteurs de lumière au niveau de l'extrémité proximale.

7. Appareil selon la revendication 6, dans lequel la deuxième pluralité d'émetteurs de lumière est en outre configurée pour transmettre une lumière depuis l'extrémité distale vers l'extrémité proximale.

8. Appareil selon la revendication 6, comprenant de plus un microprocesseur couplé au détecteur de lumière pour interpréter la lumière reçue en provenance de la deuxième pluralité d'émetteurs de lumière au niveau de l'extrémité proximale.

9. Appareil selon la revendication 7, dans lequel la première pluralité d'émetteurs de lumière est capable de transmettre une lumière comprenant au moins une lumière parmi une lumière visible, une lumière infrarouge, une lumière bleue, et une lumière laser.

10. Appareil selon la revendication 7, dans lequel le corps allongé comprend :
une partie rigide, et
au moins une partie sensiblement souple.

11. Appareil selon la revendication 10, dans lequel la au moins une partie sensiblement souple est constituée d'un tronçon jointif.

12. Appareil selon la revendication 11, dans lequel le tronçon jointif prend dans une position neutre un angle d'environ 15 à 20 degrés par rapport à un axe de la partie rigide du corps allongé.

13. Appareil selon la revendication 6, dans lequel :
la première pluralité d'émetteurs de lumière comprend une première pluralité de fibres optiques ; et la deuxième pluralité d'émetteurs de lumière comprend une deuxième pluralité de fibres optiques.

14. Appareil selon la revendication 13, dans lequel la première pluralité d'émetteurs de lumière comprend au moins un élément modifiant la lumière capable d'influencer la lumière transmise vers l'extrémité distale.

15. Appareil selon la revendication 6, comprenant de plus :
une troisième pluralité d'émetteurs de lumière s'étendant depuis l'extrémité proximale jusqu'à l'extrémité distale, la troisième pluralité d'émetteurs de lumière étant configurée pour recevoir une lumière provenant de l'extrémité distale et diriger la lumière vers l'extrémité proximale ; et
une caméra couplée pour recevoir une lumière provenant d'au moins une partie de la troisième pluralité émetteurs de lumière.

16. Appareil selon la revendication 15, dans lequel une lumière transmise à partir de l'extrémité distale vers l'extrémité proximale par la deuxième pluralité d'émetteurs de lumière est sensiblement la même qu'une lumière reçue par la troisième pluralité d'émetteurs de lumière, et dirigée par celle-ci vers l'extrémité proximale.

17. Appareil selon la revendication 6, dans lequel l'appareil comprend de plus une caméra qui est couplée pour recevoir une lumière provenant de la deuxième pluralité d'émetteurs de lumière.

18. Appareil selon la revendication 6, comprenant de plus un diviseur de faisceau couplé à une ou plusieurs parmi (a) au moins une partie de la première pluralité d'émetteurs de lumière et (b) au moins une partie de la deuxième pluralité d'émetteurs de lumière.

19. Appareil selon la revendication 18, dans lequel le détecteur de lumière est couplé pour recevoir une lumière provenant d'une partie de la deuxième pluralité d'émetteurs de lumière.

20. Appareil selon la revendication 18, comprenant de plus une caméra couplée pour recevoir une lumière provenant d'au moins une partie de la deuxième pluralité d'émetteurs de lumière.

21. Appareil selon la revendication 1, dans lequel la pièce à main (20) est reliée à une unité de base d'énergie électromagnétique (30), la pièce à main comprenant une partie allongée (22) couplée pour recevoir une énergie électromagnétique concentrée et une énergie électromagnétique supplémentaire en provenance d'un connecteur (40) qui peut être connecté à l'unité de base d'énergie électromagnétique (30) dans lequel l'embout de pièce à main (45) est formé sous la forme d'un prolongement de la partie allongée et le premier miroir (425) est disposé dans un voisinage général entre des extrémités opposées de la pièce à main et de la partie allongée.

22. Appareil selon la revendication 21, dans lequel :
le premier miroir (425) peut recevoir une énergie électromagnétique supplémentaire en provenance d'un ou plusieurs conduits et diriger l'énergie électromagnétique supplémentaire vers un ou plusieurs guides d'onde d'embout de l'embout de pièce à main (45), les guides d'onde d'embout dirigeant l'énergie électromagnétique supplémentaire vers la cible ; et
le second miroir (420) peut recevoir une énergie électromagnétique concentrée en provenance d'un conduit d'énergie électromagnétique et diriger l'énergie électromagnétique concentrée vers un embout de fibre de l'embout de pièce à main, l'embout de fibre dirigeant l'énergie électromagnétique concentrée vers la cible.

23. Appareil selon la revendication 21, dans lequel :
la partie allongée est couplée pour recevoir de l'eau de pulvérisation en provenance du connecteur, et
la partie allongée comprend une ligne d'eau de pulvérisation couplée pour recevoir de l'eau de pulvérisation et pour acheminer l'eau de pulvérisation à travers au moins une partie de la partie allongée jusqu'à l'embout de pièce à main (45).

24. Appareil selon la revendication 21, dans lequel l'énergie électromagnétique supplémentaire comprend une lumière d'éclairage et une lumière d'excitation qui sont toutes deux acheminées à travers au moins une partie de la partie allongée jusqu'à l'embout de pièce à main (45).

25. Appareil selon la revendication 24, dans lequel la lumière d'éclairage et la lumière d'excitation sont acheminées à travers au moins une partie de la partie allongée par l'intermédiaire d'une fibre d'éclairage et d'une fibre d'excitation.

26. Appareil selon la revendication 24, dans lequel la pièce à main allongée reçoit de plus de l'eau de pulvérisation en provenance du connecteur.

27. Appareil selon la revendication 26 dans lequel de l'air est en outre fourni à la pièce à main allongée par l'intermédiaire d'une ligne d'air de pulvérisation.

28. Appareil selon la revendication 21, dans lequel la partie allongée comprend :
une fibre d'énergie électromagnétique (39) pouvant recevoir une énergie électromagnétique concentrée provenant du connecteur ;
une fibre d'éclairage (36) pouvant recevoir une partie de l'énergie électromagnétique supplémentaire sous la forme de lumière d'éclairage ;
une fibre d'excitation (37) pouvant recevoir une autre partie de l'énergie électromagnétique supplémentaire sous la forme de lumière d'excitation ; et
une fibre de rétroaction (38) capable de recevoir une lumière de rétroaction provenant de l'embout de pièce à main (45).

29. Appareil selon la revendication 28, le premier miroir (425) étant disposé dans l'embout de pièce à main (45) et pouvant diriger la lumière supplémentaire sous forme de lumière d'éclairage dans une pluralité de guides d'onde d'embout, le second miroir (420) étant disposé dans l'embout de pièce à main (45) et pouvant diriger l'énergie électromagnétique concentrée dans un embout de fibre, et les guides d'onde d'embout pouvant recevoir une lumière d'éclairage et d'excitation provenant du premier miroir (45) et diriger la lumière d'éclairage vers la cible et recevoir une lumière réfléchie provenant de la cible et diriger la lumière réfléchie vers le premier miroir (425).

30. Appareil selon la revendication 29, comprenant de plus au moins une fibre de rétroaction disposée dans l'embout de pièce à main (45) et dans la partie allongée, la fibre de rétroaction pouvant recevoir une lumière réfléchie provenant du premier miroir (425) et diriger la lumière de rétroaction vers le connecteur.

31. Appareil selon la revendication 30, dans lequel l'unité de base d'énergie électromagnétique comprend un photodétecteur capable de recevoir la lumière de rétroaction provenant du connecteur et de fournir un affichage de rétroaction conformément à un parmi un état d'erreur et un état d'erreur potentielle dans des composants optiques de l'appareil.

32. Appareil selon la revendication 21, comprenant de plus :
une première tuyauterie disposée dans la partie allongée et l'embout de pièce à main (45), la première tuyauterie pouvant recevoir de l'air provenant du connecteur, et une seconde tuyauterie disposée dans la partie allongée et l'embout de pièce à main (45), la seconde tuyauterie pouvant recevoir de l'eau provenant du connecteur.

33. Appareil selon la revendication 32, comprenant de plus une ou plusieurs chambres de mélange, chaque chambre de mélange ayant deux entrées et une sortie de fluide, les deux entrées comprenant :
une première entrée pouvant recevoir de l'air provenant de la première tuyauterie ; et
une seconde entrée capable de recevoir de l'eau provenant de la seconde tuyauterie, l'air et l'eau étant mélangés dans la chambre de mélange, et un mélange d'air et d'eau est expulsé à partir de la sortie de fluide.

34. Appareil selon la revendication 32, les une ou plusieurs chambres de mélange comprenant trois chambres de mélange.

35. Appareil selon la revendication un, l'appareil comprenant de plus :
une unité de base d'énergie électromagnétique (30) ;
un connecteur (40) qui est connecté à l'unité de base d'énergie électromagnétique ;
un conduit (35) qui est connecté au connecteur et à la pièce à main, la pièce à main pouvant recevoir les énergies électromagnétiques d'infrarouge concentrée et visible moins concentrée sous la forme d'une ou plusieurs parmi une lumière d'éclairage et une lumière d'excitation, et du fluide provenant de l'unité de base d'énergie électromagnétique.

36. Appareil selon la revendication 35, dans lequel le fluide est constitué d'eau de pulvérisation, d'air de pulvérisation, et d'air de refroidissement.

37. Appareil selon la revendication 35, dans lequel la pièce à main (20) comprend un embout de pièce à main (45), l'embout de pièce à main comprenant :
une pluralité de miroirs ;
un embout de fibre ; et
une pluralité de guides d'onde d'embout, pouvant recevoir une ou plusieurs parmi une énergie électromagnétique, une lumière d'éclairage et une lumière d'excitation, et diriger les une ou plusieurs parmi une énergie électromagnétique, une lumière d'éclairage et une lumière d'excitation vers une cible.

38. Appareil selon la revendication 37, dans lequel l'embout de pièce à main (45) comprend un boîtier ayant disposés dans celui-ci la pluralité de guides d'onde d'embout, l'embout de fibre, et une pluralité de sorties de fluide.

39. Appareil selon la revendication 38, dans lequel la pluralité de guides d'onde d'embout et la pluralité de sorties de fluide sont disposées circulairement autour de l'embout de fibre.

40. Appareil selon la revendication 39, dans lequel :
la pluralité de guides d'onde d'embout comprend neuf guides d'ondes d'embout séparés par environ quarante degrés ; et
la pluralité de sorties de fluide comprend trois sorties de fluide séparées par environ cent vingt degrés.

41. Appareil selon la revendication 40, dans lequel :
les neuf guides d'onde d'embout sont disposés par rapport à une référence zéro, à quarante, quatre-vingt, cent vingt, cent soixante , deux cents, deux cent quarante, deux cent quatre-vingt, et trois cent vingt degrés ; et
les trois sorties de fluide sont disposées par rapport à la référence à cent, deux cent vingt, et trois cent quarante degrés°.

42. Appareil selon la revendication 37, dans lequel l'embout de pièce à main (45) comprend un boîtier ayant disposé dans celui-ci un matériau transparent pouvant transmettre une lumière.

43. Appareil selon la revendication 42, dans lequel le matériau transparent est constitué d'un matériau parmi une matière plastique, du saphir, et du quartz.

44. Appareil selon la revendication 1, la pièce à main (20) comprenant :
une fibre de puissance s'étendant depuis l'extrémité proximale jusqu'à l'extrémité distale ;
une pluralité de premières fibres optiques agencées concentriquement autour de la fibre de puissance et s'étendant depuis l'extrémité proximale jusqu'à l'extrémité distale, la pluralité de premières fibres optiques pouvant recevoir un premier type d'énergie électromagnétique provenant de l'extrémité proximale et émettre le premier type d'énergie électromagnétique au niveau de l'extrémité distale ; et
une pluralité de deuxièmes fibres optiques agencées concentriquement autour de la fibre de puissance et s'étendant depuis l'extrémité proximale vers l'extrémité distale, la pluralité de deuxièmes fibres optiques pouvant recevoir un second type d'énergie électromagnétique provenant de l'extrémité distale et diriger le deuxième type d'énergie électromagnétique vers l'extrémité proximale.

45. Appareil selon la revendication 44, comprenant de plus :
une pluralité de troisièmes fibres optiques s'étendant depuis l'extrémité proximale jusqu'à l'extrémité distale, la pluralité de troisièmes fibres optiques pouvant recevoir un troisième type d'énergie électromagnétique provenant de l'extrémité distale et diriger le troisième type d'énergie électromagnétique vers l'extrémité proximale et;
une caméra couplée pour recevoir le troisième type d'énergie électromagnétique provenant d'au moins une partie de la pluralité des troisièmes fibres optiques.

46. Appareil selon la revendication 45, dans lequel le deuxième type d'énergie électromagnétique est sensiblement le même que le troisième type d'énergie électromagnétique.

47. Appareil selon la revendication 44, comprenant de plus un détecteur d'énergie électromagnétique couplé pour recevoir une énergie électromagnétique provenant d'au moins une partie de la pluralité de deuxièmes fibres optiques.

48. Appareil selon la revendication 47, dans lequel le détecteur d'énergie électromagnétique comprend une caméra couplée pour recevoir une énergie électromagnétique provenant d'au moins une partie de la pluralité de deuxièmes fibres optiques.

49. Appareil selon la revendication 47, dans lequel :
le détecteur d'énergie électromagnétique est couplé pour recevoir le deuxième type d'énergie électromagnétique en provenance d'au moins une partie de la pluralité de deuxièmes fibres optiques ; et
la pièce à main (20) comprend de plus une caméra qui est couplée pour recevoir le deuxième type d'énergie provenant d'au moins une partie de la pluralité de deuxièmes fibres optiques.

50. Appareil selon la revendication 44, dans lequel la pluralité de premières fibres optiques peut recevoir une énergie électromagnétique comprenant une ou plusieurs lumières parmi une lumière visible, une lumière infrarouge, une lumière bleue, et une lumière laser.

51. Appareil selon la revendication 44, dans lequel l'appareil comprend au moins un élément modifiant une lumière, capable d'influencer une transmission d'énergie électromagnétique par la pluralité de premières fibres optiques.

52. Appareil selon la revendication 51, dans lequel le au moins un élément modifiant une lumière comprend au moins une fibre optique.

53. Appareil selon la revendication 52, dans lequel le au moins un filtre optique est structuré pour convertir une lumière bleue en lumière blanche.

54. Appareil selon la revendication 44, comprenant de plus un diviseur de faisceau couplé à une ou plusieurs parmi (a) au moins une partie de la pluralité de premières fibres optiques et (b) au moins une partie de la pluralité de deuxièmes fibres.

55. Procédé d'analyse de lumière de rétroaction utilisant l'appareil selon la revendication 1, surveillant ainsi l'intégrité de composants optiques, le procédé comprend :
recevoir une lumière de rétroaction dans la pièce à main (20) ;
produire un signal électrique conforme à la lumière de rétroaction ; et
fournir une indication d'erreur lorsque le signal électrique dépasse un seuil prédéterminé.

56. Procédé selon la revendication 55, dans lequel la fourniture d'une indication d'erreur consiste à produire un affichage sur un écran d'une unité de base d'énergie électromagnétique.
